# EUROPEAN PATENT APPLICATION

(11) **EP 2 666 761 A1**
(43) Date of publication of application: **27.11.2013**
(21) Application number: 11848331.2
(22) Date of filing: 19.12.2011
(51) Int. Cl.: C07C 43/164, A61K 31/075, A61P 3/00

(54) **HYDROXYTYROSOL ETHERS**

(30) Priority: 17.12.2010 ES 201031878
(71) Applicant: Fundacio Imim, 08003 Barcelona (ES); Fundación Instituto Mediterráneo Para El Avance De La Biotecnologia Y La Investigacion Sanitaria (IMABIS), 29010 Malaga (ES); Consejo Superior De Investigaciones Científicas, 28006 Madrid (ES)
(72) Inventor: DE LA TORRE FORNELL, Rafael, 08003 Barcelona (ES); FARRÉ ALBALADEJO, Magín, 08003 Barcelona (ES); COVAS PLANELLS, María Isabel, 08003 Barcelona (ES); FITÓ COLOMER, Montserrat, 08003 Barcelona (ES); ALMEIDA COTRIM, Bruno, 08003 Barcelona (ES); RODRÍGUEZ DE FONSECA, Fernando, 29010 Málaga (ES); DECARA DEL OLMO, Juan Manuel, 29010 Málaga (ES); ROMERO CUEVAS, Miguel, 29010 Málaga (ES); JOGLAR TAMARGO, Jesús, 08034 Barcelona (ES); CLAPÉS SABORIT, Pedro, 08034 Barcelona (ES)
(74) Representative: Elzaburu Marquez, Alberto
(86) International application number: PCT/ES2011/070880
(87) International publication number: WO 2012/080555

(57) **Abstract**

Hydroxytyrosol ethers. The invention relates to hydroxytyrosol ethers derived from fatty alcohols and phenolic compounds of olive oil and the salts, solvates and hydrates thereof, which have an affinity for type 1 cannabinoid receptors (CB₁) and which can: prevent the oxidation of low-density lipoprotein (LDL); and modulate the actions regulated by said receptor, such as inducing satiety, controlling intake and reducing body fat.

## Description

The present invention relates to a new series of ethers derived from fatty alcohols and phenolic compounds of olive oil and the salts, solvates and hydrates thereof showing affinity for cannabinoid receptors type 1 (CB₁) and are capable of preventing low-density lipoprotein (LDL) oxidation. These compounds can modulate the actions regulated by the mentioned receptor, such as inducing satiety and controlling intake and reducing body fat.

### Prior Art

The endocannabinoid system is made up of cannabinoid receptors, endogenous ligands (endocannabinoids) and the enzyme systems necessary for their biosynthesis and degradation (Annu. Rev. Pharmacol. 2006 46:101). Up until now two types of cannabinoid receptors have been identified: CB₁ and CB₂. The two cannabinoid receptors are G-protein-coupled receptors and through this protein they modulate the activity of adenylate cyclases (AC) and mitogen-activated protein kinases (MAPK), and intracellular events leading to regulation in the expression of various genes. The activation of CB₁ receptors also regulates potassium and voltage-dependent Ca²⁺ channels. CB₁ receptors are distributed throughout the central nervous system and in other organs such as adipose tissue, endocrine pancreas, muscle, lungs, liver and kidneys, whereas CB₂ receptors are mainly expressed in the immune system and hematopoietic cells (Nat. Rev. Drug Discov. 2004 3:771).

The endocannabinoid system seems to be related to a wide range of physiological and pathological conditions at the neurological, psychiatric, and cardiovascular level, to the development of cancer, reproductive disorders and to eating disorders. Better knowledge of endocannabinoid biosynthesis pathways and the mechanisms of regulating said pathways at the cellular level are considered the top priorities in cannabinoid research (Nat. Rev. Drug Discov. 2004 3:771).

All the endocannabinoid compounds described up until now are derived from fatty acids with polar heads. This polar head can be bound to the fatty acid through an amide-type bond (anandamide, *N*-arachidonoyl-dopamine (NADA), *N*-oleoyl-dopamine (OLDA)), ester (2-arachidonoylglycerol, 2-AG), or ether (noladin) (Nat. Rev. Drug Discov. 2004 3:771).

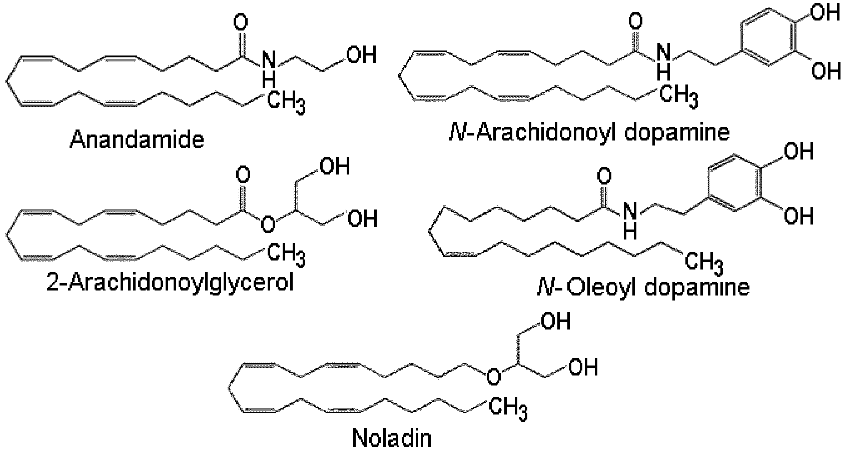

### Chemical structure of some endocannabinoids

Within the endocannabinoid system, the CB₁ receptor has been the therapeutic target that initially received the most attention in research for the treatment of obesity. It is well known that cannabinoid agonist substances increase appetite, and so it was postulated that by blocking this receptor food intake could be reduced, leading to weight loss. Rimonabant, also known as SR141716 or Acomplia®, was the first CB₁ antagonist to be described and one of the first to be clinically studied for the treatment of obesity (Annu. Rev. Pharmacol. 2006 46:101, Nat. Rev. Drug Discov. 2004 3:771). The clinical trials called RIO (Rimonabant In Obesity) (Lancet 2005 365:1389; J. Am. Med. Assoc. 2006 295:761; Lancet 2006 368:1160) showed the efficacy of Rimonabant as an anti-obesity agent. Unfortunately data from the clinical studies has associated the chronic use of Rimonabant with an increase in depression, anxiety and an increase in suicidal tendencies (Lancet 2007 370:1706; Lancet 20 2008 371:556; Lancet 2008 371:555). So in October 2008, the European Medicines Agency decided to temporarily suspend Rimonabant.

Hydroxytyrosol is a phenolic compound naturally occurring in virgin olive oil. It is a potent *in vitro* inhibitor of low-density lipoprotein (LDL) oxidation, being capable of interrupting peroxidation chain reactions (Atherosclerosis 1995 117:25, 1995). Hydroxytyrosol is also a natural metabolite of dopamine and is structurally similar to it (J. Agr. Food Chem. 2001 49:2480; J. Agr. Food Chem. 2003 51:7170).

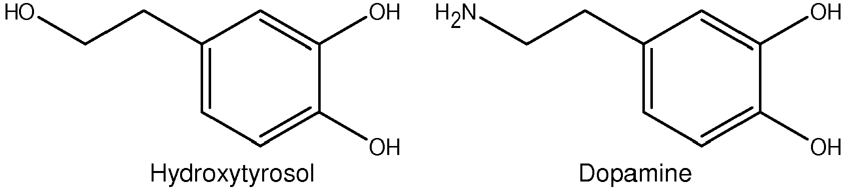

### Structures of dopamine and hydroxytyrosol

Some ester and ether derivatives of hydroxytyrosol with fatty acids have already been synthesized in an attempt to increase their bioavailability, and in both cases, the hydroxytyrosol derivatives showed antioxidant activity in lipid matrices that was equivalent to or greater than free hydroxytyrosol (J. Agr. Food Chem. 2006, 54, 3779; Molecules 2009 14:1762). In the case of ethers, only the preparation of saturated derivatives is reported because the described pathway (Molecules 2009 14:1762) does not allow synthesis of unsaturated derivatives because hydrogenation in the last step is necessary to deprotect the catechol group. In any case, none of the synthesized derivatives (ethers or esters) was evaluated as an inhibitor of food intake or as a ligand of the CB₁ receptor.

Dyslipidemias are lipid metabolism alterations, with their subsequent alteration of concentrations of blood lipids (e.g., cholesterol and triglycerides) and lipoproteins: low-density lipoproteins (LDL), very low-density lipoproteins (VLDL) and intermediate-density lipoproteins (IDL). Cholesterol molecules are usually transported bound to LDLs. An increase in concentrations of LDL-cholesterol is directly related to the risk of coronary disease. A smaller percentage of cholesterol molecules are transported through high-density lipoproteins, HDL, the primary function of which is to extract cholesterol deposited on arterial walls and transport it to the liver for elimination through the intestine. It has been described that a high level of HDL cholesterol is associated with the reduction of the risk of coronary disease. Therefore, in the treatment of dyslipidemias it is equally important to reduce concentrations of LDL-cholesterol as it is to increase concentrations of HDL-cholesterol (Am. J. Med. 1977 62:707; N. Engl J. Med. 1991 325:373; Ann. Intern. Med. 1979 90:85). Fibrate derivatives are being used today at the clinical level to control dyslipidemias (Am J Med. 2009 122:962), giving rise to different therapies with derivatives such as the clofibrate and fenofibrate (WO2007047880 2007; WO2007047724 2007), which bind to the PPAR-alpha receptor and regulate different transcription factors involved in some of the previously described processes (Curr. Atheroscler. Rep. 2000 2:327). In addition to the treatment of dyslipidemias, dual PPAR-alpha/gamma agonist agents with potential use for the treatment of type 2 diabetes have been described (J. Med. Chem. 2004 30 47:4118).

Coronary disease is the main cause of death in industrialized countries. Oxidation of the lipids present in low-density lipoproteins (LDL) is a marker of the development of arteriosclerosis and coronary disease (Cell 2001 104:503). It is postulated that the excessive production of reactive oxygen species (ROS) is involved in the pathogenesis of arteriosclerosis and hypertension (Physiol. Rev. 2002 82:47). LDL oxidation by ROS is one of the first events in the development of the disease. Arteriosclerosis can be considered as a type of chronic inflammation resulting from the interaction between modified lipoproteins, macrophages, T-cells and natural cell elements of the arterial wall. The inflammatory process can lead to the development of complex lesions or plaques. Rupture of the plaques and thrombosis leads to myocardial infarction (Cell 2001 104:503).

The present invention proposes a new synthetic route for synthesizing ethers derived from hydroxytyrosol with unsaturated fatty alcohols such as oleic and linoleic alcohols. Taking into account the chemical structure of endocannabinoids (e.g., OLDA and NADA) and the structural similarity of hydroxytyrosol with dopamine, the present invention proposes a possible activity of these compounds on intake that may be derived from interaction with the CB₁ receptor. The fact that these compounds, in addition to regulating satiety, can have a protective effect on LDL oxidation because both the hydroxytyrosol ethers and the hydroxytyrosol esters were characterized as potent antioxidants in lipid matrices (J. Agr. Food Chem. 2006 54:3779; Molecules 2009 14: 1762), may be interesting because this activity is related to a reduction of the risk of cardiovascular problems which are often associated with obesity.

### Description of the Invention

The invention relates to a new class of molecules, specifically ethers derived from unsaturated fatty alcohols conjugated with phenolic compounds from olive oil as ligands of the CB₁ receptor and inhibitors of LDL oxidation, and it also relates to the method of preparation and the use thereof.

The present invention describes fatty alcohol derivatives with phenolic compounds from olive oil for the treatment of eating disorders. These compounds can be used for preparing a medicament for inducing satiety and controlling intake, modulating body fat and regulating lipid metabolism, as well as preparing a medicament for the treatment of diabetes, obesity, metabolic syndrome and cardiovascular diseases.

Therefore, the present invention relates to a new family of compounds derived from fatty acids with phenolic compounds from olive oil of formula I having clear inhibitory activity on the appetite and showing affinity for the CB₁ receptor. The fundamental role that the aforementioned receptors have in a wide range of diseases and conditions, particularly those related to eating, is known.

One aspect of the present invention relates to a compound of formula I: 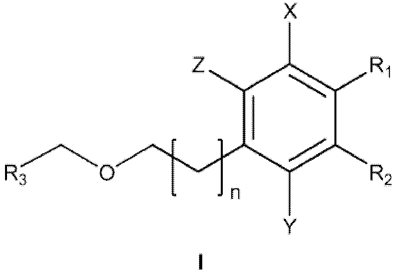 or a salt thereof, where:
each independent X, Y and Z represents hydrogen, halogen, C₁-C₆ alkyl or C₂-C₆ alkenyl, where the C₁-C₆ alkyl and C₂-C₆ alkenyl groups are optionally substituted with one or more R₄ groups;
n represents from 1 to 4;
R₁ and R₂ each independently represents hydrogen or -OR₅;
R₃ represents C₈-C₃₀ alkenyl or C₈-C₃₀ alkynyl;
each R₄ independently represents halogen, -NO₂, -CN, -C₁-C₄ alkoxyl, -NR₆R₆, -NR₆COR₆, -NR₆CONR₆R₆, -NR₆CO₂R₆, -NR₆SO₂R₆, -OR₆,-OCOR₆, -OCONR₆R₆, -OCO₂R₆, -SR₆, -SOR₆, -SO₂R₆, -SO₂NR₆R₆, -SO₂NR₆COR₆, -COR₆, -CO₂R₆ or -CONR₆R₆;
each R₅ independently represents hydrogen or C₁-C₆ alkyl;
or when R₁ and R₂ simultaneously represent -OR₅, the two R₅ groups are optionally bound forming a group of formula -O-W-O-;
W represents C₁-C₄ alkylenyl optionally substituted with one or more C₁-C₄ alkyl, =O, =NR₆ or =S; and
each R₆ independently represents hydrogen or C₁-C₄ alkyl,
on the proviso that the compound (9*Z*,12*Z*)-1-(2-(3,4-methylenedioxyphenyl)ethoxy)-octadeca-9,12-diene is excluded.

Another aspect of the invention relates to a pharmaceutical composition comprising at least one of the compounds of formula I as defined in claims 1 to 16, or a salt thereof, and at least one pharmaceutically acceptable carrier, adjuvant and/or vehicle.

In another embodiment, the invention relates to a pharmaceutical composition as previously defined, further comprising another active ingredient.

Another aspect of the invention relates to the use of a compound of formula II: 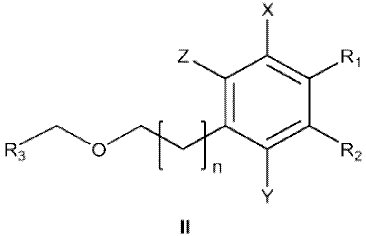 or a salt thereof, where:
each independent X, Y and Z represents hydrogen, halogen, C₁-C₆ alkyl or C₂-C₆ alkenyl, where the C₁-C₆ alkyl and C₂-C₆ alkenyl groups are optionally substituted with one or more R₄ groups;
n represents from 1 to 4;
R₁ and R₂ each independently represents hydrogen or -OR₅;
R₃ represents C₈-C₃₀ alkenyl or C₈-C₃₀ alkynyl;
each R₄ independently represents halogen, -NO₂, -CN, -C₁-C₄ alkoxyl, -NR₆R₆, -NR₆COR₆, -NR₆CONR₆R₆, -NR₆CO₂R₆, -NR₆SO₂R₆, -OR₆,-OCOR₆, -OCONR₆R₆, -OCO₂R₆, -SR₆, -SOR₆, -SO₂R₆, -SO₂NR₆R₆, -SO₂NR₆COR₆, -COR₆, -CO₂R₆ or -CONR₆R₆;
each R₅ independently represents hydrogen or C₁-C₆ alkyl;
or when R₁ and R₂ simultaneously represent -OR₅, the two R₅ groups are optionally bound forming a group of formula -O-W-O-;
W represents C₁-C₄ alkylenyl optionally substituted with one or more C₁-C₄ alkyl, =O, =NR₆ or =S; and
each R₆ independently represents hydrogen or C₁-C₄ alkyl,
for manufacturing a medicament.

Another aspect of the invention relates to the use of a compound of formula II as previously defined for manufacturing a medicament for the treatment and/or prevention of a disease mediated by the cannabinoid CB₁ receptor and/or by inhibition of LDL oxidation; preferably for the treatment and/or prevention of a disease selected from an eating disorder; more preferably for the treatment and/or prevention of a disease selected from obesity, lipid dysfunction, diabetes, cardiovascular diseases and metabolic syndrome; and even more preferably to reduce subcutaneous fat and/or to induce satiety and control intake.

Another aspect of the invention relates to the use of a compound of formula II as previously defined for manufacturing a medicament for the treatment and/or prevention of LDL oxidation, preferably for the treatment or prevention of arteriosclerosis.

Another aspect of the invention relates to the use of a compound of formula II as previously defined for manufacturing a medicament for the treatment and/or prevention of LDL oxidation.

Another aspect of the invention relates to the use of a compound of formula II as previously defined for manufacturing a medicament for the treatment and/or prevention of a disease associated with LDL oxidation, and preferably for manufacturing a medicament for the treatment and/or prevention of arteriosclerosis.

Another aspect of the invention relates to the use of a compound of formula II as previously defined for manufacturing a medicament for the treatment and/or prevention of arteriosclerosis.

Another aspect of the invention relates to a compound of formula II: 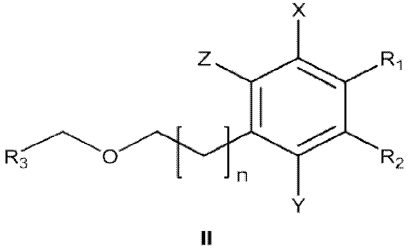 or a salt thereof, where:
each independent X, Y and Z represents hydrogen, halogen, C₁-C₆ alkyl or C₂-C₆ alkenyl, where the C₁-C₆ alkyl and C₂-C₆ alkenyl groups are optionally substituted with one or more R₄ groups;
n represents from 1 to 4;
R₁ and R₂ each independently represents hydrogen or -OR₅;
R₃ represents C₈-C₃₀ alkenyl or C₈-C₃₀ alkynyl;
each R₄ independently represents halogen, -NO₂, -CN, -C₁-C₄ alkoxyl, -NR₆R₆, -NR₆COR₆, -NR₆CONR₆R₆, -NR₆CO₂R₆, -NR₆SO₂R₆, -OR₆,-OCOR₆, -OCONR₆R₆, -OCO₂R₆, -SR₆, -SOR₆, -SO₂R₆, -SO₂NR₆R₆, -SO₂NR₆COR₆, -COR₆, -CO₂R₆ or -CONR₆R₆;
each R₅ independently represents hydrogen or C₁-C₆ alkyl;
or when R₁ and R₂ simultaneously represent -OR₅, the two R₅ groups are optionally bound forming a group of formula -O-W-O-;
W represents C₁-C₄ alkylenyl optionally substituted with one or more C₁- C₄ alkyl, =O, =NR₆ or =S; and
each R₆ independently represents hydrogen or C₁-C₄ alkyl,
for use in therapy.

Another aspect of the invention relates to a method for preparing a compound of formula I as previously defined comprising:
a) reacting a compound of formula IV with a compound of formula V: 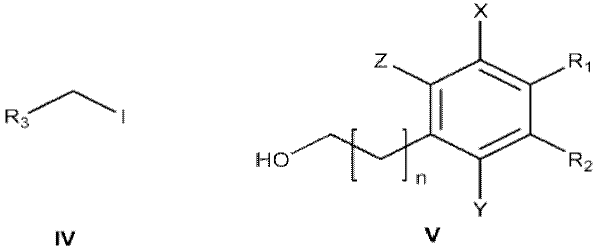 where X, Y, Z, R₁, R₂, R₃ and n have the previously described meaning; or
b) converting a compound of formula I into another compound of formula I in one or several steps.

In the preceding definitions, the term C₁-C₆ alkyl, as a group or part of a group, means a straight or branched chain alkyl group containing from 1 to 6 carbon atoms. Examples include, among others, methyl, ethyl, propyl, isopropyl, butyl, isobutyl, sec-butyl and *tert*-butyl, pentyl and hexyl groups.

The term C₁-C₄ alkyl, as a group or part of a group, means a straight or branched chain alkyl group containing from 1 to 4 carbon atoms and includes methyl, ethyl, propyl, isopropyl, butyl, isobutyl, *sec*-butyl and *tert*-butyl groups.

The term C₁-C₄ alkylenyl refers to a divalent analog of a straight or branched chain C₁-C₄ alkyl group containing from 1 to 4 carbon atoms and includes methylene, ethylene, propylene, isopropylene, butylene, isobutylene, *sec*-butylene and *tert-*butylene groups.

A C₂-C₆ alkenyl group means a straight or branched alkyl chain containing from 2 to 6 carbon atoms and further containing one or two double bonds.

Examples include, among others, ethenyl, 1 -propenyl, 2-propenyl, isopropenyl, 1-butenyl, 2-butenyl, 3-butenyl, 1,3-butadienyl, 1-pentenyl, 2-pentenyl, 2,4-pentadienyl, 1-hexenyl, 2-hexenyl, 3-hexenyl and 2,4-hexadienyl groups.

A C₈-C₃₀ alkenyl group means a straight or branched alkyl chain containing from 8 to 30 carbon atoms and further containing one or more double bonds, preferably one or two. Examples include, among others, 9-octadecenyl and 9,12-octadecadienyl groups.

A C₈-C₃₀ alkynyl group means a straight or branched alkyl chain containing from 8 to 30 carbon atoms and further containing one or more triple bonds, preferably one or two.

A C₁-C₄ alkoxy group, as a group or part of a group, means a -OC₁-C₄ alkyl group, where the C₁-C₄ alkyl part has the same previously described meaning. Examples include methoxy, ethoxy, propoxy, isopropoxy, butoxy, isobutoxy, *sec*-butoxy and *tert-*butoxy.

A radical halogen or its abbreviation halo means fluorine, chlorine, bromine or iodine.

The expression "optionally substituted with one or more" means the possibility of a group being substituted with one or more, preferably with 1, 2, 3 or 4, substituents, more preferably with 1, 2 or 3 substituents, and even more preferably with 1 or 2 substituents, provided that said group has enough available positions that can be substituted. If present, said substituents can be the same or different and can be located on any available position.

When there are two or more groups with the same numbering in a definition of a substituent (for example -NR₆R_{6,} etc.), this does not mean that they have to be identical. Each of them is selected independently from the list of possible meanings given for said group and can therefore be the same or different.

Throughout the present description, the term "treatment" refers to eliminating, reducing or decreasing the cause or the effects of a disease. For the purposes of this invention, treatment includes, but is not limited to, alleviating,, decreasing or eliminating one or more symptoms of the disease; reducing the stage of the disease, stabilizing (i.e., not worsening) the state of the disease, delaying or slowing down the progression of the disease, alleviating or improving the state of the disease and putting said disease into remission (either completely or partially).

As it is used herein, the term "prevention" refers to preventing the onset of the disease presenting in a patient who is predisposed to or has the risk factors of said disease but still shows no symptoms. Prevention also includes preventing the recurrence of a disease in a subject who has previously suffered said disease.

The invention therefore relates to the compounds of formula I as they have been previously defined.

In another embodiment, the invention relates to a compound of formula I as previously defined where X, Y and Z independently represent hydrogen, halogen or C₁-C₆ alkyl, where the C₁-C₆ alkyl group is optionally substituted with one or more R₄ groups.

In another embodiment, the invention relates to a compound of formula I as previously defined where X and Y independently represent hydrogen.

In another embodiment, the invention relates to a compound of formula I as previously defined where Z represents hydrogen.

In another embodiment, the invention relates to a compound of formula I as previously defined where n represents 1.

In another embodiment, the invention relates to a compound of formula I as previously defined where R₁ and R₂ each independently represents hydrogen.

In another embodiment, the invention relates to a compound of formula I as previously defined where:
R₁ represents hydrogen; and
R₂ represents -OR₅.

In another embodiment, the invention relates to a compound of formula I as previously defined where:
R₁ represents -OR₅; and
R₂ represents hydrogen.

In another embodiment, the invention relates to a compound of formula I as previously defined where R₁ and R₂ independently represent -OR₅.

In another embodiment, the invention relates to a compound of formula I as previously defined where R₁ and R₂ simultaneously represent -OR₅.

In another embodiment, the invention relates to a compound of formula I as previously defined where when R₁ and R₂ simultaneously represent -OR₅, the two R₅ groups are bound forming a group of formula -O-W-O-.

In another embodiment, the invention relates to a compound of formula I as previously defined where W represents C₁-C₄ alkylenyl optionally substituted with one or more C₁-C₄ alkyl.

In another embodiment, the invention relates to a compound of formula III: 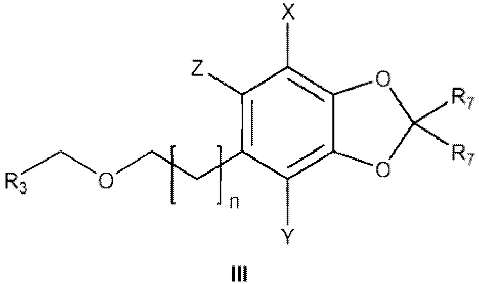 where:
R₃ has the meaning defined previously for a compound of formula I; and
each R₇ independently represents C₁-C₄ alkyl, preferably methyl.

In another embodiment, the invention relates to a compound of formula I as previously defined where:
R₁ represents -OR₅;
R₂ represents hydrogen; and
R₃ represents C₈-C₃₀ alkenyl.

In another embodiment, the invention relates to a compound of formula I as previously defined where:
R₁ represents hydrogen;
R₂ represents -OR₅; and
R₃ represents C₈-C₃₀ alkenyl.

In another embodiment, the invention relates to a compound of formula I as previously defined where each R₄ independently represents halogen, -C₁-C₄ alkoxyl, -NR₆R₆, -OR₆, -SR₆, -SOR₆,-SO₂R₆, -COR₆, -CO₂R₆ or -CONR₆R₆; preferably halogen, -C₁-C₄ alkoxyl, -NR₆R₆, -OR₆, -SR₆ or -COR₆.

In another embodiment, the invention relates to a compound of formula I as previously defined where each R₅ independently represents hydrogen.

Likewise, the present invention covers all the possible combinations of the particular and preferred embodiments described above.

In another embodiment, the invention relates to the compounds of formula I producing over 50% inhibition of CB₁ activity at 10 µM and more preferably at 1 µM in a ligand-receptor assay for the CB₁ receptor such as the one described in Example 13.

In another embodiment, the invention relates to a compound of formula I selected from the list of compounds described in Examples 1 to 12.

In another embodiment, the invention relates to a compound of formula I selected from:
(*Z*)-1-(2-phenylethoxy)octadec-9-ene;
(*Z*)-1-(2-(4-hydroxyphenyl)ethoxy)octadec-9-ene;
(*Z*)-1-(2-(3,4-methylenedioxyphenyl)ethoxy)octadec-9-ene;
(*Z*)-1-(2-(3,4-dihydroxyphenyl)ethoxy)octadec-9-ene;
(9*Z*,12*Z*)-1-(2-phenylethoxy)-octadeca-9,12-diene;
(9*Z*,12*Z*)-1-(2-(4-hydroxyphenyl)ethoxy)-octadeca-9,12-diene; and
(9*Z*,12*Z*)-1-(2-(3,4-dihydroxyphenyl)ethoxy)-octadeca-9,12-diene.

The compounds of formula I can exist in different physical forms, i.e., in amorphous form and crystalline forms. The compounds of the present invention can also have the capacity to crystallize into more than one form, a characteristic which is known as polymorphism. Polymorphs can be differentiated from one another by several physical properties that are well known by those skilled in the art, such as for example their x-ray diffractograms, melting points or solubility. All the physical forms of the compounds of formula I, including all their polymorphic forms ("polymorphs"), are included within the scope of the present invention.

The compounds of the present invention represented by formula I can include isomers, depending on the presence of multiple bonds, including optical isomers or enantiomers, depending on the presence of chiral centers. Individual isomers, enantiomers or diastereoisomers and the mixtures thereof fall within the scope of the present invention, i.e., the term isomer also refers to any mixture of isomers, such as diastereomeric isomers, racemic isomers, etc., even their optically active isomers or mixtures with different proportions of said isomers. Individual enantiomers or diastereoisomers, as well as their mixtures, can be separated by means of conventional techniques.

Likewise, prodrugs of the compounds of formula I are within the scope of this invention. As it is used herein, the term "prodrug" includes any compound derived from a compound of formula I, including the following non-limiting examples: esters (including carboxylic acid esters, amino acid esters, phosphate esters, sulfonate esters of metal salts, etc.), carbamates, amides, etc., which when administered to an individual can be converted directly or indirectly into said compound of formula I in the mentioned individual. Advantageously, said derivative is a compound that increases the bioavailability of the compound of formula I when administered to an individual or that enhances the release of the compound of formula I in a biological compartment. The nature of said derivative is not critical provided that it can be administered to an individual and provides the compound of formula I in a biological compartment of the individual. The preparation of said prodrug can be carried out by means of conventional methods known by the persons skilled in the art.

The compounds of the invention can be in crystalline form as free compounds or as solvates. In this sense, as it is used herein the term "solvate" includes both pharmaceutically acceptable solvates, i.e., solvates of the compound of formula I that can be used in the elaboration of a medicament, and pharmaceutically unacceptable solvates, which can be useful in the preparation of pharmaceutically acceptable solvates or salts. The nature of the pharmaceutically acceptable solvate is not critical provided that it is pharmaceutically acceptable. In a particular embodiment, the solvate is a hydrate. The solvates can be obtained by conventional solvation methods known by the persons skilled in the art.

For their application in therapy, the compounds of formula I, the salts, prodrugs or solvates thereof will preferably be in a pharmaceutically acceptable or substantially pure form, i.e., having a pharmaceutically acceptable level of purity, excluding normal pharmaceutical additives such as diluents and carriers, and not including material considered toxic at normal dosage levels. The levels of purity for the active ingredient are preferably greater than 50%, more preferably greater than 70%, and still more preferably greater than 90%. In a preferred embodiment, the levels of purity are greater than 95% of the compound of formula I or of the salts, solvates or prodrugs thereof.

The pharmaceutically acceptable adjuvants and vehicles that can be used in said compositions are the adjuvants and vehicles known by the persons skilled in the art and commonly used in the elaboration of therapeutic compositions.

In the sense used in this description, the expression "therapeutically effective amount" refers to the amount of the agent or compound capable of performing the therapeutic action determined by its pharmacological properties, calculated for producing the desired effect, and it will generally be determined by, among others, the characteristics inherent to the compounds, including age, condition of the patient, seriousness of the alteration or disorder, and the administration route and frequency.

The compounds described in the present invention, the salts, prodrugs and/or solvates thereof, as well as the pharmaceutical compositions containing them can be used together with other additional drugs or active ingredients to provide a combination therapy. Said additional drugs can be part of the same pharmaceutical composition or, alternatively, they can be provided in the form of a separate composition for administration that is or is not simultaneous to the administration of the pharmaceutical composition comprising a compound of formula I, or a salt, prodrug or solvate thereof.

In a preferred embodiment of the present invention, the pharmaceutical compositions are suitable for oral administration, in solid or liquid forms. The possible forms for oral administration are tablets, capsules, syrups or solutions and can contain conventional excipients known in the pharmaceutical field, such as aggregating agents (e.g., syrup, acacia, gelatin, sorbitol, tragacanth or polyvinylpyrrolidone), fillers (e.g., lactose, sugar, corn starch, calcium phosphate, sorbitol or glycine), disintegrants (e.g., starch, polyvinylpyrrolidone or microcrystalline cellulose) or a pharmaceutically acceptable surfactant, such as sodium lauryl sulfate.

The compositions for oral administration can be prepared using conventional Galenic pharmacy methods, such as mixing and dispersion. The tablets can be coated following methods known in the pharmaceutical industry.

The pharmaceutical compositions can be adapted for parenteral administration, such as sterile solutions, suspensions, or lyophilisates of the products of the invention, using the suitable dose. Suitable excipients, such as pH buffering agents or surfactants, can be used.

The previously mentioned formulations can be prepared using conventional methods, such as those described in the pharmacopoeias of different countries and in other reference texts.

The compounds or compositions of the present invention can be administered by means of any suitable method, such as intravenous infusion and oral, intraperitoneal or intravenous routes. Oral administration is preferred due to convenience for patients and the chronic nature of the diseases to be treated.

The administered amount of a compound of the present invention will depend on the relative efficacy of the chosen compound, the seriousness of the disease to be treated and the weight of the patient. However, the compounds of this invention will be administered once or more times a day, for example 1, 2, 3 or 4 times daily, with a total dose between 0.1 and 1000 mg/Kg/day. It is important to take into account that it may be necessary to introduce variations in the dose, depending on the age and on the condition of the patient, and it may be necessary to modify the administration route.

The compounds and compositions of the present invention can be used together with other medicaments in combined therapies. The other drugs can be part of the same composition or part of another different composition for administration at the same time or at different times.

The compounds of formula I can be obtained following the methods described below. As will be obvious for a person skilled in the art, the precise method used for preparing a given compound can vary according to its chemical structure. In some the methods described below, it may also be necessary or convenient to protect the reactive or labile groups by means of conventional protecting groups. Both the nature of said protecting groups and the methods for their introduction and elimination are well known and are part of the state of the art (see, for example, Wuts P.G.M and Greene T.W., "Greene's Protective Groups in Organic Synthesis", John Wiley & Sons, 4th edition, 2006).

Unless otherwise indicated, in the methods described below the meanings of the different substituents are the meanings described previously in relation to a compound of formula I.

The compounds of formula I can generally be obtained by the following method:
- Obtaining the compound of formula IV from the compound of formula VI through reaction thereof with iodine, triphenylphosphine and imidazole (Scheme 1). 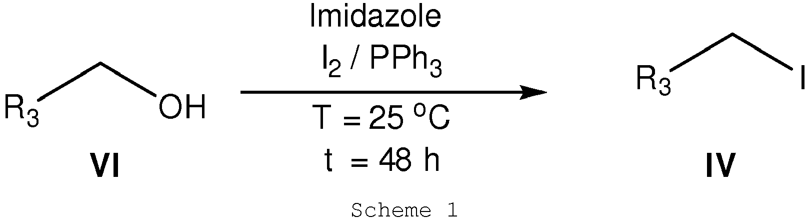 where R₃ has the previously described meaning.
- conjugation between a compound of formula IV and a compound of formula V, giving rise to the compound of formula I using a two-phase water/toluene system with a quaternary ammonium salt-type phase transfer agent, such as, for example, but not exclusively tetrabutylammonium bromide (nBu)₄NBr (Scheme 2). 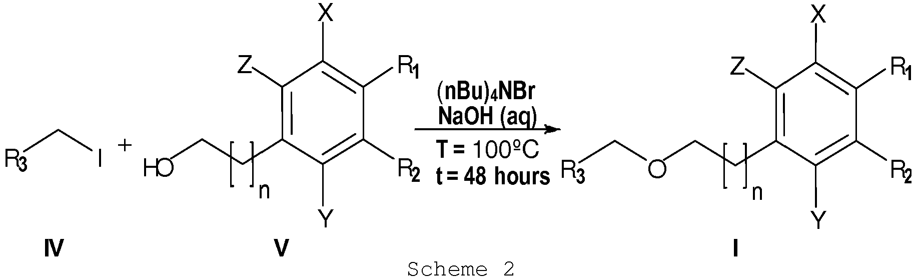 where X, Y, Z, R₁, R₂, R₃ and n have the previously described meaning for a compound of formula I.
- separating the phases;
- extracting the organic phase twice with distilled water and once with brine;
- treating the organic phase with anhydrous sodium sulfate and evaporating the solvent;
- purifying the compound of formula I by silica gel FLASH column chromatography.

Alternatively, a compound of formula V where R₁ and R₂ represent -OC(CH₃)₂O- is synthesized in two steps from the protection of the compound of formula VII producing the compound of formula VIII and subsequently reducing this compound with NaBH₄/I₂ in a manner similar to that described by Gambacorta et al. (J. Agr. Food Chem. 2007 55:3386, incorporated herein by reference) (Scheme 3). 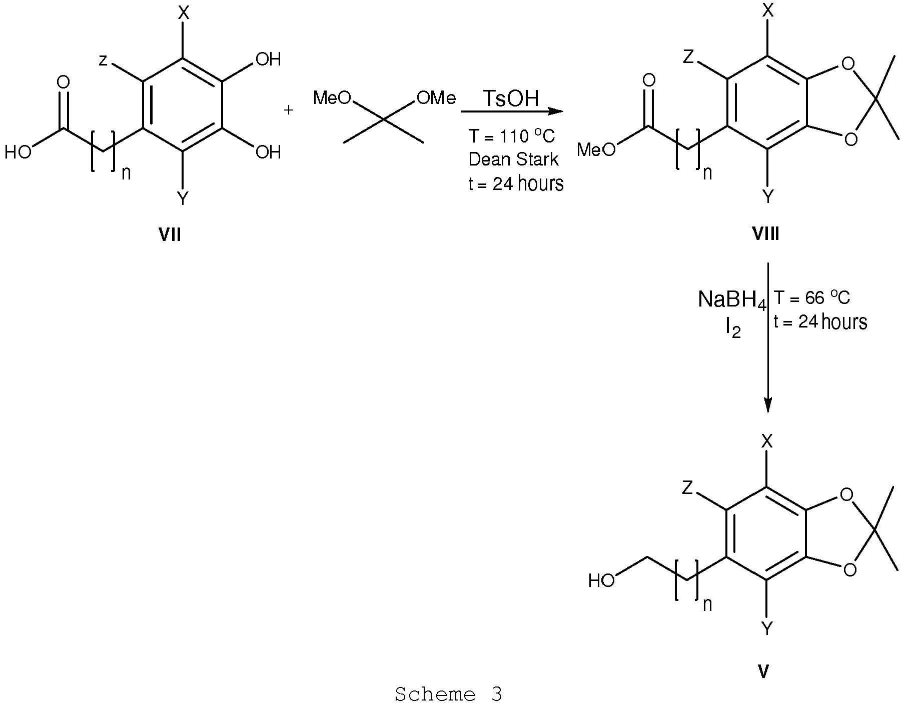 where X, Y, Z and n have the previously described meaning for a compound of formula I.

Alternatively, a compound of formula V where R₁ represents -O-CH₂-O-CH₃, R₂ represents hydrogen or an -OR₅ group and R₅ represents a C₁-C₆ alkyl group is synthesized from the protection of the compound of formula IX with, for example, MOMCl (Tetrahedron Lett. 1978 7:661) (Scheme 4). 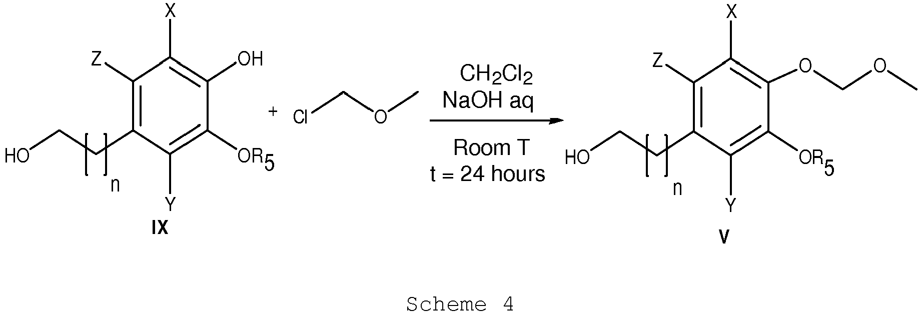 where X, Y, Z, n and R₅ have the previously described meaning for a compound of formula I.

Alternatively, the deprotection of the catechol group can be carried out on a compound of formula I where R₁ and R₂ represent -OC(CH₃)₂O- through acid hydrolysis, such as for example with hydrochloric acid in aqueous medium for obtaining a compound of formula I where R₁ and R₂ represent a hydroxyl group (Scheme 5). 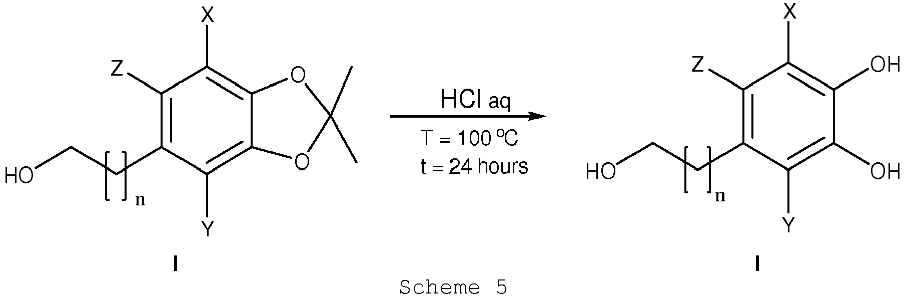 where X, Y, Z and n have the previously described meaning for a compound of formula I.

Alternatively, for a compound of formula I where R₁ represents -O-CH₂-O-CH₃, R₂ represents hydrogen or an -OR₅ group and R₅ represents a C₁-C₆ alkyl group, the deprotection of the phenol group can be performed through acid hydrolysis, such as with hydrochloric acid in a mixture of water/isopropanol, for example, for obtaining the compound of formula I where R₁ represents a hydroxyl group, R₂ represents hydrogen or an -OR₅ group and R₅ represents a C₁-C₆ alkyl group (Scheme 6). 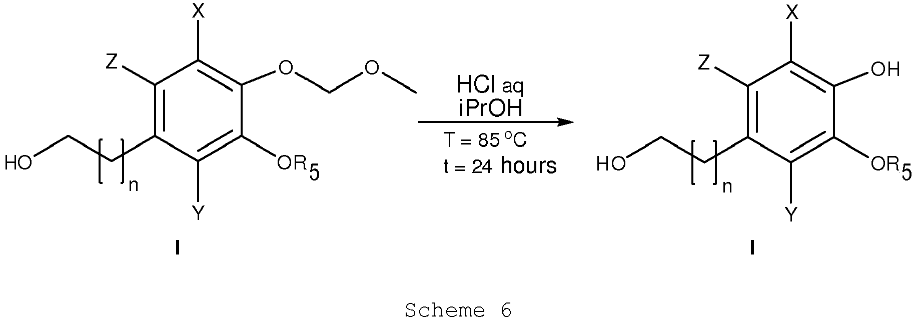 where X, Y, Z, n and R₅ have the previously described meaning for a compound of formula I.

Throughout the description and the claims, the word "comprises" and its variants do not intend to exclude other technical features, additives, components or steps. For the persons skilled in the art, other objects, advantages and features of the invention will be inferred partly from the description and partly from putting the invention into practice. The following examples and drawings are provided by way of illustration and do not intend to limit the present invention.

### Description of the Drawings

Figure 1 shows the acute intake experiment with (*Z*)-1-(2-phenylethoxy)octadec-9-ene (Example 3).
Figure 2 shows the acute intake experiment with (*Z*)-1-(2-(4-hydroxyphenyl)ethoxy)octadec-9-ene (Example 11).
Figure 3 shows the acute intake experiment with (*Z*)-1-(2-(3,4-methylenedioxyphenyl)ethoxy)octadec-9-ene (Example 2).
Figure 4 shows the acute intake experiment with (*Z*)-1-(2-(3,4-dihydroxyphenyl)ethoxy)octadec-9-ene (Example 9).
Figure 5 shows the acute intake experiment with (9*Z*,12*Z*)-1-(2-phenylethoxy)-octadeca-9,12-diene (Example 6).
Figure 6 shows the acute intake experiment with (9*Z*,12*Z*)-1-(2-(4-hydroxyphenyl)ethoxy)-octadeca-9,12-diene (Example 12).
Figure 7 shows the acute intake experiment with (9*Z*,12*Z*)-1-(2-(3,4-methylenedioxyphenyl)ethoxy)-octadeca-9,12-diene (Example 5).
Figure 8 shows the acute intake experiment with (9*Z*,12*Z*)-1-(2-(3,4-dihydroxyphenyl)ethoxy)-octadeca-9,12-diene (Example 10).
Figure 9 shows the ligand-receptor assay with (*Z*)-1-(2-phenylethoxy)octadec-9-ene (Example 3).
Figure 10 shows the ligand-receptor assay with (*Z*)-1-(2-(3,4-dihydroxyphenyl)ethoxy)octadec-9-ene (Example 9).
Figure 11 shows the ligand-receptor assay with (9*Z*,12*Z*)-1-(2-(3,4-methylenedioxyphenyl)ethoxy)-octadeca-9,12-diene (Example 5).
Figure 12 shows the ligand-receptor assay with (9*Z*,12*Z*)-1-(2-(3,4-dihydroxyphenyl)ethoxy)-octadeca-9,12-diene (Example 10). Examples

A series of examples provided at all times to illustrate the synthesis of some particular compounds of the present invention and to give an example of the general methods are shown below. According to the foregoing, the following section of examples in no way intends to limit the scope of the invention contemplated in the present specification.

In this specification, the symbols and conventions used in these methods, schemes and examples are consistent with those used in the International System and contemporary scientific literature, for example, the Journal of Medicinal Chemistry. Unless otherwise indicated, all the starting materials were obtained from commercial suppliers and were used without additional purification. Specifically, the following abbreviations can be used in the examples and throughout the entire specification: g (grams); mg (milligrams); kg (kilograms); µg (micrograms); L (liters); mL (milliliters); µL (microliters); mmol (millimoles); mol (moles); °C (degrees Celsius); Hz (hertz); MHz (megahertz); δ (chemical shift); s (singlet); d (doublet); t (triplet); q (quartet); m (multiplet); NMR (nuclear magnetic resonance); M (molar); DMSO (dimethyl sulfoxide); PBS (pH-regulating phosphate buffer solution), MOMCl (chloromethyl methyl ether).

Unless indicated, all the reagents and solvents used were obtained from commercial suppliers and were used without any prior purification. All the ¹H and ¹³C NMR analyses were performed with Varian Anova 500 and Varian Mercury 400 spectrometers. The progress of all the reactions was monitored by TLC (thin-layer chromatography) in aluminum sheets with a 0.25 mm thick layer of silica gel 60 (HF-254, Merck).

### Reference example 1

### 3,4-(dimethylmethylenedioxy)phenylacetic acid methyl ester

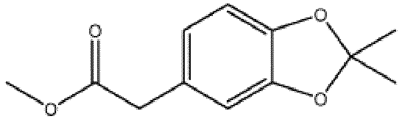

Dihydroxyphenylacetic acid (11.9 mmol), *p*-toluenesulfonic acid (1.2 mmol), 2,2-dimethoxypropane (69.2 mmol) and toluene (30 mL) were added in a round-bottom flask equipped with a Dean-Stark apparatus and a magnetic stirrer. The mixture was stirred for 24 hours under reflux temperature. The organic phase was washed twice with distilled water and once with brine and evaporated to dryness. The product was purified by flash chromatography column using ethyl acetate/hexane as an eluent.

The product was obtained as a transparent oil with a yield of 94%. ¹H NMR (400 MHz, CDCl₃) δ ppm 1.66 (s, 6H); 3.52 (s, 2H); 3.69 (s, 3H); 6.64-6.71 (m, 3H). ¹³C NMR (101 MHz, CDCl₃) δ ppm 25.85; 40.80; 52.01; 108.04; 109.44; 117.94; 121.70; 146.61; 147.55; 172.25. IR (KBr): ν cm⁻¹= 2890, 2952, 1740, 1498, 1255, 981, 838.

### Reference example 2

### 2-(3,4-(dimethylmethylenedioxy)phenyl)ethanol

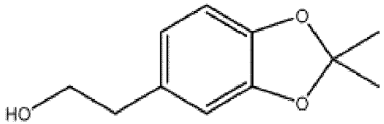

3,4-(dimethylmethylenedioxy)phenylacetic acid methyl ester (5.6 mmol), NaBH₄ (13.2 mmol) and THF (100 mL) were added in a round-bottom flask equipped with a magnetic stirrer and cooled to 4°C, and iodine (5.6 mmol) dissolved in THF (20 mL) was slowly added to this mixture. The mixture was stirred for 24 hours under reflux temperature. The reaction was monitored by TLC.

THF was removed under reduced pressure and the residue dissolved in ethyl acetate. The organic phase was washed twice with distilled water and once with brine and evaporated to dryness. The product was purified by flash chromatography column using ethyl acetate/hexane as an eluent.

The product was obtained as a yellow oil with a yield of 72%. ¹H NMR (500 MHz, CDCl₃) δ ppm 1.69 (s, 6H); 2.80 (t, *J* = 6.47 Hz, 2H); 3.83 (t, *J* = 6.07 Hz, 2H); 6. 74-6.62 (m, 3H). ¹³C NMR (101 MHz, CDCl₃) δ ppm 25.83; 38.85; 63.75; 108.08; 109.06; 117. 71; 121.23; 131.36; 145.99; 147.57. IR (KBr) : ν cm⁻¹ = 3370, 2989, 2937, 2872, 1739, 1498, 1445, 1255, 1046, 981, 839, 807.

### Reference example 3

### 2-(4-(methoxymethoxy)phenyl)ethanol

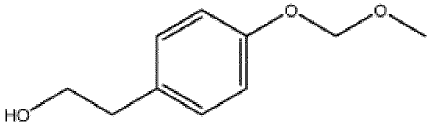

Tyrosol (0.26 mmol), MOMCl (3.51 mmol), tetrabutylammonium bromide (0.15 mmol), dichloromethane (6 mL) and 30% aq. NaOH (6 mL) were added in a round-bottom flask equipped with a magnetic stirrer. The mixture was stirred for 24 hours at room temperature. The reaction was monitored by TLC. The organic phase was separated and washed twice with distilled water and once with brine and evaporated to dryness. The product was purified by flash chromatography column using ethyl acetate/hexane as an eluent.

The product was obtained as a white solid with a yield of 65%. ¹H NMR (400 MHz, CDCl₃) δ ppm 2.80 (t, *J* = 6.57 Hz, 2H); 3.47 (s, 3H); 3.80 (t, *J* = 6.60 Hz, 2H); 5.15 (s, 2H); 6.99 (d, *J* = 8.48 Hz, 2H); 7.14 (d, *J* = 8.40 Hz, 2H). ¹³C NMR (101 MHz, CDCl₃) δ ppm 38.24; 55.85; 63.66; 94.39; 116.33; 129.92; 131.77; 155.75. IR (KBr): ν cm⁻¹= 3404, 2937, 2827, 1612, 1513, 1233, 1199, 1152, 1110, 1079, 1008, 922, 825.

### A. Synthesis of the compounds of the invention

### Example 1

### (Z)-1-(2-(3,4-(dimethylmethylenedioxy)phenyl)ethoxy)octadec-9-ene

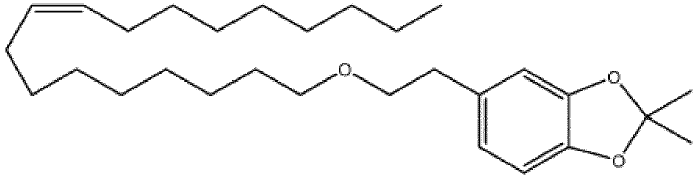

2-(3,4-(dimethylmethylenedioxy)phenyl)ethanol (0.6 mmol), oleyl iodide (1.32 mmol), tetrabutylammonium bromide (0.16 mmol), 30% aq. KOH (10 mL) and toluene (10 mL) were added in a round-bottom flask equipped with a magnetic stirrer and the mixture was heated under reflux for 48 hours. The reaction was monitored by TLC. The organic phase was separated and washed twice with distilled water and once with brine and evaporated to dryness. The product was purified by flash chromatography column using ethyl acetate/hexane as an eluent.

The product was obtained as a transparent oil with a yield of 30%. ¹H NMR (400 MHz, CDCl₃) δ ppm 0.88 (t, *J* = 6.21 Hz, 3H); 1.20-1.39 (m, 22H); 1.47-1.62 (m, 2H); 1.66 (s, 6H); 1.96-2.07 (m, 4H); 2.79 (t, *J* = 7.28 Hz, 2H); 3.42 (t, *J* = 6.72 Hz, 2H); 3.57 (t, *J* = 7.32 Hz, 2H); 5.30-5.40 (m, 2H); 6.57-6.68 (m, 3H). ¹³C NMR (101 MHz, CDCl₃) δ ppm 14.12; 22.68; 25.83; 26.16; 27.20; 29.25; 29.32; 29.47; 29.49; 29.52; 29.72; 29.76; 31.90; 36.05; 71.07; 72.06; 107.90; 109.14; 117.52; 121.02; 129.84; 129.91; 132.09; 145.69; 147.30. IR (KBr): ν = 2924, 2835, 1499, 1252, 1234, 1112, 980, 842.

### Example 2

### (2)-1-(2-(3,4-methylenedioxyphenyl)ethoxy)octadec-9-ene

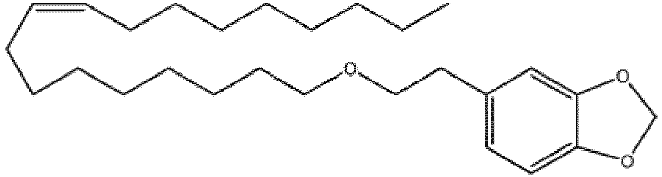

This compound was synthesized as described in Example 1 using the same starting reagents and the same molar amounts substituting 2-(3,4-(dimethylmethylenedioxy)phenyl)ethanol with 2-(3,4-methylenedioxyphenyl)ethanol.

The product was obtained as a transparent oil with a yield of 35%. ¹H NMR (400 MHz, CDCl₃) δ ppm 0.88 (t, *J* = 5.79 Hz, 3H); 1.19-1.40 (m, 22H); 1.49-1.65 (m, 2H); 1.95-2.07 (m, 4H); 2.80 (t, *J* = 7.12 Hz, 2H); 3.57 (t, *J* = 7.16 Hz, 2H); 3.42 (t, *J* = 6.63 Hz, 2H); 5.35 (m, 2H); 5.92 (s, 2H); 6.66 (d, *J* = 7.99 Hz, 1H); 6.73 (m, 2H). ¹³C NMR (101 MHz, CDCl₃) δ ppm 14.12; 22.68; 26.16; 29.19; 29.25; 29.32; 29.45; 29.49; 29.52; 29.70; 29.76; 31.90; 36.04; 71.08; 71.93; 100.74; 108.10; 109.35; 121.66; 129.83; 129.91; 132.88; 145.81; 147.45. IR (KBr) : ν cm⁻¹ = 2924, 2854, 1506, 1490, 1246, 1112, 1042, 940, 639.

### Example 3

### (Z)-1-(2-phenylethoxy)octadec-9-ene

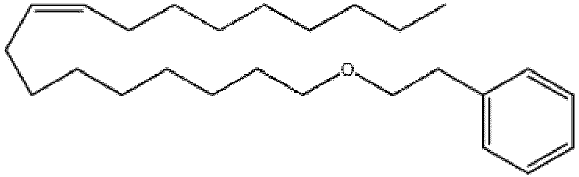

This compound was synthesized as described in Example 1 using the same starting reagents and the same molar amounts substituting 2-(3,4-(dimethylmethylenedioxy)phenyl)ethanol with 2-phenylethanol.

The product was obtained as a transparent oil with a yield of 20%. ¹H NMR (400 MHz, CDCl₃) δ ppm 0.89 (t, *J* = 6.09 Hz, 3H); 1.21 -1.40 (m, 22H); 1.64-1.51 (m, 2H); 2.02 (m, 4H); 2.89 (t, *J* = 7.28 Hz, 2H); 3.43 (t, *J* = 6.68 Hz, 2H); 3.63 (t, *J* = 7.34 Hz, 2H); 5.29-5.42 (m, 2H); 7.36-7.15 (m, 5H). ¹³C NMR (101 MHz, CDCl₃) δ ppm 14.12; 22.68; 26.15; 27.19; 29.24; 29.32; 29.45; 29.49; 29.52; 29.71; 29.76; 31.90; 36.37; 71.07; 71.79; 126.09; 128.27; 128.87; 129.82; 129.90; 139.03. IR (KBr) : ν cm⁻¹ = 2924, 2854, 2358, 2699, 1113, 747, 639.

### Example 4

### (9Z,12Z)-1-(2-(3,4-(dimethylmethylenedioxy)phenyl)ethoxy)octadeca-9,12-diene

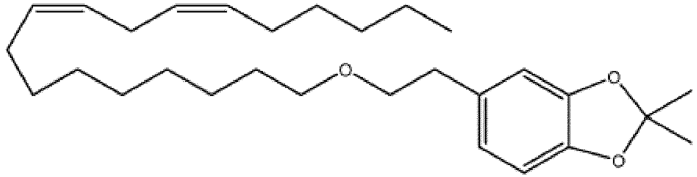

This compound was synthesized as described in Example 1 using the same starting reagents and the same molar amounts substituting oleyl iodide with linoleyl iodide.

The product was obtained as a transparent oil with a yield of 53%. ¹H NMR (400 MHz, CDCl₃) δ ppm 0.89 (t, *J* = 6.51 Hz, 3H); 1.22-1.42 (m, 20H); 1.50-1.62 (m 2H); 1.66 (s, 6H); 2.04 (m, 4H); 2.85-2.72 (m, 4H); 3.42 (t, *J* = 6.51 Hz, 2H); 3.57 (t, *J* = 7.31 Hz, 2H); 5.48-5.26 (m, 4H); 6.58-6.67 (m, 3H). ¹³C NMR (101 MHz, CDCl₃) δ ppm 14.07; 22.57; 25.61; 25.82; 26.16; 26.18; 27.18; 27.21; 29.25; 29.34; 29.45; 29.49; 29.65; 29.71; 29.77; 31.51; 36.04; 70.95; 71.04; 72.05; 107.89; 109.13; 117.49; 121.00; 127.93; 130.11; 130.16; 132.08; 145.69; 147.29. IR (KBr): ν cm⁻¹ = 3009, 2927, 2855, 1499, 1253, 1234, 1113, 980.

### Example 5

### (9Z,12Z)-1-(2-(3,4-methylenedioxyphenyl)ethoxy)-octadeca-9,12-diene

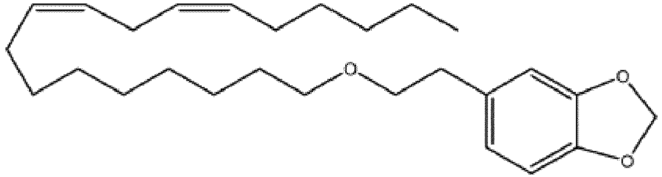

This compound was synthesized as described in Example 1 using the same starting reagents and the same molar amounts substituting 2-(3,4-(dimethylmethylenedioxy)phenyl)ethanol with 2-(3,4-methylenedioxyphenyl)ethanol and oleyl iodide with linoleyl iodide.

The product was obtained as a transparent oil with a yield of 23%. ¹H NMR (500 MHz, CDCl₃) δ ppm 0.89 (t, *J* = 6.42 Hz, 3H); 1.22-1.42 (m, 20H); 1.49-1.64 (m, 2H); 1.98-2.12 (m, 2H); 2.72-2.85 (m, 4H); 3.42 (t, *J* = 6.65 Hz, 2H); 3.57 (t, *J* = 7.21 Hz, 2H); 5.26-5.46 (m, 4H); 5.92 (s, 2H); 6.62-6.78 (m, 3H). ¹³C NMR (101 MHz, CDCl₃) δ ppm 14.07; 22.61; 25.61; 26.15; 27.19; 29.23; 29.25; 29.34; 29.40; 29.44; 29.49; 29.66; 29.69; 31.52; 36.04; 64.64; 71.08; 71.93; 100.74; 108.11; 109.35; 121.67; 127.94; 130.18; 132.89; 145.81; 147.45. IR (KBr): ν cm⁻¹ = 2927, 2855, 1741, 1489, 1246, 1113, 1043.

### Example 6

### (9Z,12Z)-1-(2-phenylethoxy)-octadeca-9,12-diene

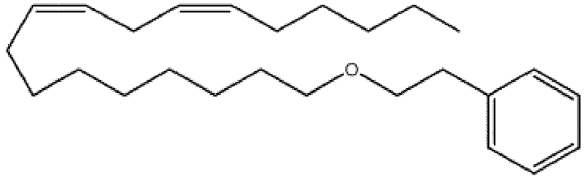

This compound was synthesized as described in Example 1 using the same starting reagents and the same molar amounts substituting 2-(3,4-(dimethylmethylenedioxy)phenyl)ethanol with 2-phenylethanol and oleyl iodide with linoleyl iodide.

The product was obtained as a transparent oil with a yield of 27.0%. ¹H NMR (400 MHz, CDCl₃) δ ppm 0.88 (t, *J* = 6.75 Hz, 3H); 1.15-1.44 (m, 20H); 1.49-1.67 (m, 2H); 1.99-2.10 (m, 4H); 2.78 (t, *J* = 6.08 Hz, 2H); 2.89 (t, *J* = 7.29 Hz, 2H); 3.43 (t, *J* = 6.68 Hz, 2H); 3.62 (t, *J* = 7.35 Hz, 2H); 5.27-5.46 (m, 4H); 7.40-7.14 (m, 5H).¹³C NMR (101 MHz, CDCl₃) δ ppm 14.06; 22.56; 25.60; 26.14; 27.18; 29.24; 29.33; 29.43; 29.47; 29.65; 29.69; 31.51; 36.35; 71.06; 71.78; 126.09; 127.90; 127.93; 128.27; 128.87; 130.12; 130.17; 139.03. IR (KBr): ν cm⁻¹ = 3009, 2927, 2855, 2361, 1738, 1455, 1115, 698.

### Example 7

### (Z)-1-(2-(4-(methoxymethoxy)phenyl)ethoxy)octadec-9-ene

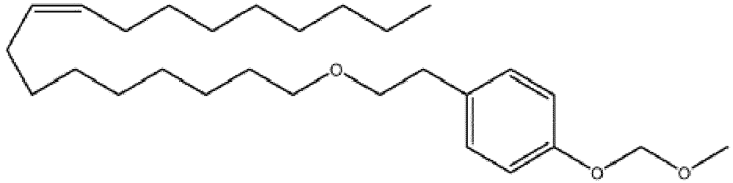

This compound was synthesized as described in Example 1 using the same starting reagents and the same molar amounts substituting 2-(3,4-(dimethylmethylenedioxy)phenyl)ethanol with 2-(4-(methoxymethoxy)phenyl)ethanol.

The product was obtained as a transparent oil with a yield of 30%. ¹H NMR (400 MHz, CDCl₃) δ ppm 0.88 (t, *J* = 6.45 Hz, 3H); 1.20-1.41 (m, 22H); 1.48-1.68 (m, 2H); 1.93-2.11 (m, 4H); 2.83 (t, *J* = 7.27 Hz, 2H); 3.42 (t, J = 6.70 Hz, 2H); 3.47 (s, 3H); 3.58 (t, *J* = 7.34 Hz, 2H); 5.15 (s, 2H); 5.35 (m, 2H); 6.96 (d, *J* = 8.56 Hz, 2H); 7.14 (d, *J* = 8.46 Hz, 2H). ¹³C NMR (101 MHz, CDCl₃) δ ppm 14.11; 22.67; 26.15; 27.19; 29.24; 29.31; 29.45; 29.48; 29.51; 29.71; 29.74; 31.89; 35.49; 55.88; 71.06; 71.95; 94.50; 116.14; 129.82; 129.90; 132.44; 155.60. IR (KBr): ν cm⁻¹= 2925, 2853, 1613, 1511, 1465, 1233, 1156, 1114, 1080, 1010, 921.

### Example 8

### (9Z,12Z)-1-(2-(4-(methoxymethoxy)phenyl)ethoxy)octadeca-9,12-diene

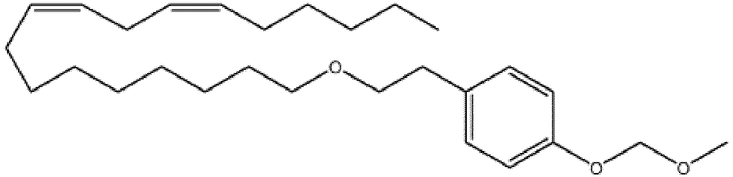

This compound was synthesized as described in Example 1 using the same starting reagents and the same molar amounts substituting 2-(3,4-(dimethylmethylenedioxy)phenyl)ethanol with 2-(4-(methoxymethoxy)phenyl)ethanol and oleyl iodide with linoleyl iodide.

The product was obtained as a transparent oil with a yield of 20.0%. ¹H NMR (400 MHz, CDCl₃) δ ppm 0.88 (t, *J* = 6.53 Hz, 3H); 1.18-1.44 (m, 20H); 1.50-1.62 (m, 2H); 1.98-2.11 (m, 4H); 2.77 (t, *J* = 6.19 Hz, 2H); 2.83 (t, *J* = 7.26 Hz, 2H); 3.42 (t, *J* = 6.67 Hz, 2H); 3.47 (s, 3H); 3.58 (t, *J* = 7.32 Hz, 1H); 5.15 (s, 1H) ; 5.27-5.46 (m, 8H); 7.14 (d, *J* = 8.23 Hz, 2H); 6. 96 (d, *J* = 8.48 Hz, 2H). ¹³C NMR (101 MHz, CDCl₃) δ ppm 14.08; 22.57; 25.61; 26.16; 27.19; 27.22; 29.25; 29.34; 29.45; 29.49; 29.65; 29.72; 31.52; 35.50; 55.91; 71.06; 71.96; 94.51; 116.15; 127.90; 127.93; 129.83; 130.13; 130.18; 132.44; 165.50. IR (KBr): ν cm⁻¹ = 3008, 2927, 2854, 1613, 1511, 1233, 1175, 1153, 1113, 1010, 828.

### Example 9

### (Z)-1-(2-(3,4-dihydroxyphenyl)ethoxy)octadec-9-ene

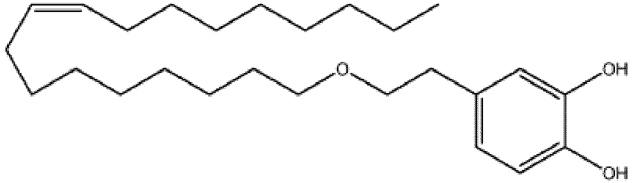

(*Z*)-1-(2-(3,4-(dimethylmethylenedioxy)phenyl)ethoxy)octadec-9-ene (0.26 mmol) and 6N aq. HC1 (5 mL) were added in a round-bottom flask equipped with a magnetic stirrer and the mixture was heated under reflux for 24 hours. The reaction was monitored by TLC. The organic phase was separated and washed twice with distilled water and once with brine and evaporated to dryness. The product was purified by flash chromatography column using ethyl acetate/hexane as an eluent.

The product was obtained as a transparent oil with a yield of 50.7%. ¹H NMR (500 MHz, CDCl₃) δ ppm 0.88 (t, *J* = 6.91 Hz, 3H); 1.18-1.38 (m, 20H); 1.51-1.62 (m, 2H); 1.97-2.04 (m, 4H); 2.76 (t, *J* = 7.14 Hz, 2H); 3.46 (t, *J* = 6.83 Hz, 1H); 3.62 (t, *J* = 7.16 Hz, 1H); 5.30-5.41 (m, 2H); 5.72 (s, 1H); 5.93 (s, 1H); 6.59-6.63 (m, 1H) ; 6.68 (d, *J* = 1.66 Hz, 1H) ; 6.74 (d, *J* = 8.03 Hz, 1H). ¹³C NMR (101 MHz, CDCl₃) δ ppm 14.12; 22.68; 26.07; 27.19; 29.23; 29.31; 29.42; 29.48; 29.51; 29.74; 29.75; 31.89; 35.41; 71.18; 71.93; 115.20; 115.85; 121.00; 129.82; 129.93; 131.57; 142.03; 143.58. IR (KBr) : ν cm⁻¹ = 3394, 2922, 2854, 1606, 1520, 1465, 1446, 1279, 1193, 1113, 1092, 810, 723.

### Example 10

### (9Z,12Z)-1-(2-(3,4-dihydroxyphenyl)ethoxy)-octadeca-9,12-diene

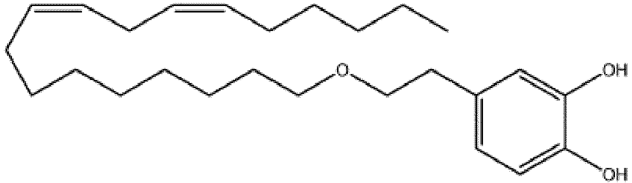

This compound was synthesized as described in Example 9 using the same starting reagents and the same molar amounts substituting (Z)-1-(2-(3,4-(dimethylmethylenedioxy)phenyl)ethoxy)octadec-9-ene with (9*Z*, 12*Z*)-1-(2-(3,4-(dimethylmethylenedioxy)phenyl)ethoxy)octadec-9,12-diene.

The product was obtained as a transparent oil with a yield of 27.0%. ¹H NMR (500 MHz, CDCl₃) δ ppm 0.89 (t, *J* = 6.96 Hz, 3H); 1.22-1.42 (m, 20H); 1.50-1.62 (m, 2H); 1.96-2.12 (m, 4H); 2.72-2.82 (m, 4H); 3.42 (t, *J* = 6.71 Hz, 2H); 3.58 (t, *J* = 7.21 Hz, 2H); 4.99 (s, 1H); 5.13 (s, 1H); 5.27-5.45 (m, 4H); 6.70-6.61 (m, 1H); 6.81-6.71 (m, 2H). ¹³C NMR (101 MHz, CDCl₃) δ ppm 14.07; 22.56; 25.60; 26.04; 27.15; 27.20; 29.23; 29.33; 29.40; 29.47; 29.63; 31.50; 35.37; 71.17; 71.93; 115.19; 115.84; 120.94; 127.89; 127.95; 130.12; 130.19; 131.44; 142.04; 143.63. IR (KBr) : ν cm⁻¹ = 3386, 3009, 2935, 2856, 1606, 1520, 1446, 1375, 1279, 1113, 811, 723.

### Example 11

### (Z)-1-(2-(4-hydroxyphenyl)ethoxy)octadec-9-ene

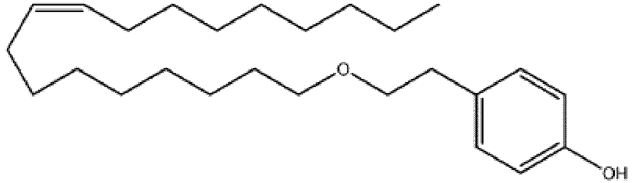

(*Z*)-1-(2-(4-(methoxymethoxy)phenyl)ethoxy)octadec-9-ene (0.17 mmol), isopropanol (5 mL) and 6N aq. HCl (2 mL) were added in a round-bottom flask equipped with a magnetic stirrer. The mixture was heated under reflux for 24 hours. The reaction was monitored by TLC. The organic phase was separated and washed twice with distilled water and once with brine and evaporated to dryness. The product was purified by flash chromatography column using ethyl acetate/hexane as an eluent.

The product was obtained as a transparent oil with a yield of 97.8%. ¹H NMR (400 MHz, CDCl₃) δ ppm 0.88 (t, *J* = 6.13 Hz, 3H); 1.20-1.42 (m, 22H); 1.50-1.65 (m, 2H); 1.94-2.09 (m, 4H); 2.82 (t, *J* = 7.24 Hz, 2H); 3.45 (t, *J* = 6.75 Hz, 2H); 3.60 (t, *J* = 7.32 Hz, 2H); 5.29-5.38 (m, 2H); 5.40 (s, 1H); 6.73 (d, *J* = 8.45 Hz, 2H); 7.07 (d, *J* = 8.32 Hz, 2H). ¹³C NMR (101 MHz, CDCl₃) δ ppm 14.12; 22.67; 26.10; 27.19; 29.23; 29.31; 29.42; 29.47; 29.51; 29.60; 29.74; 29.75; 31.89; 35.33; 71.12; 72.06; 115.16; 129.83; 129.93; 130.80; 154.05. IR (KBr): ν cm⁻¹ = 3373, 2924, 2854, 1710, 1614, 1516, 1464, 1372, 1236, 1190, 829, 722.

### Example 12

### (9Z,12Z)-1-(2-(4-hydroxyphenyl)ethoxy)-octadeca-9,12-diene

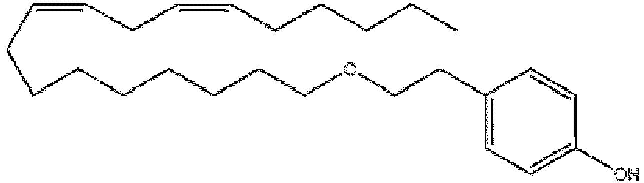

This compound was synthesized as described in Example 11 using the same starting reagents and the same molar amounts substituting (*Z*)-1-(2-(4-(methoxymethoxy)phenyl)ethoxy)octadec-9-ene with (9*Z*,12*Z*)-1-(2-(4-(methoxymethoxy)phenyl)ethoxy)octadeca-9,12-diene.

The product was obtained as a transparent oil with a yield of 89.0%. ¹H NMR (400 MHz, CDCl₃) δ ppm 0.89 (t, *J* = 6.30 Hz, 3H); 1.20-1.42 (m, 20H); 1.51 -1.63 (m, 2H); 1.99-2.09 (m, 4H); 2.73-2.87 (m, 4H); 3.44 (t, *J* = 6.75 Hz, 2H); 3.59 (t, *J* = 7.33 Hz, 2H); 5.26-5.46 (m, Hz, 4H); 6.74 (d, *J* = 8.46 Hz, 2H); 7.07 (d, *J* = 8.43 Hz, 2H). ¹³C NMR (101 MHz, CDCl₃) δ ppm 14.08; 22.56; 25.60; 26.10; 27.18; 27.21; 29.23; 29.33; 29.42; 29.47; 29.62; 29.64; 31.51; 35.35; 71.09; 72.06; 115.15; 127.89; 127.93; 129.93; 130.12; 130.18; 130.83; 154.05. IR (KBr): ν cm⁻¹ = 3361, 2927, 2855, 1615, 1516, 1458, 1228, 1111, 829.

### B. Biological activity

### Example 13

### Ligand-receptor assay

The ligand-receptor assay for the CB₁ receptor evaluates the capacity of the synthesized compounds to displace [³H] SR141716 (known ligand with affinity for CB₁ receptor) in a homogenized rat cerebellum.

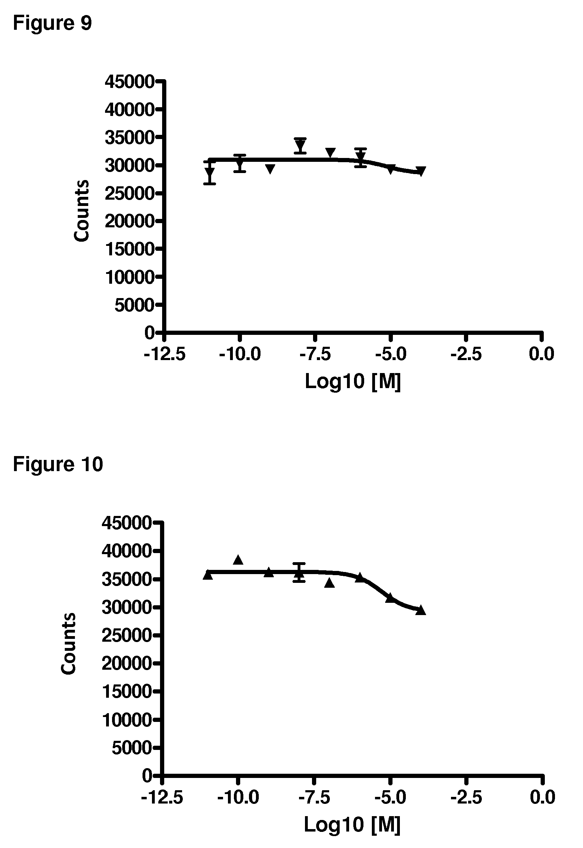

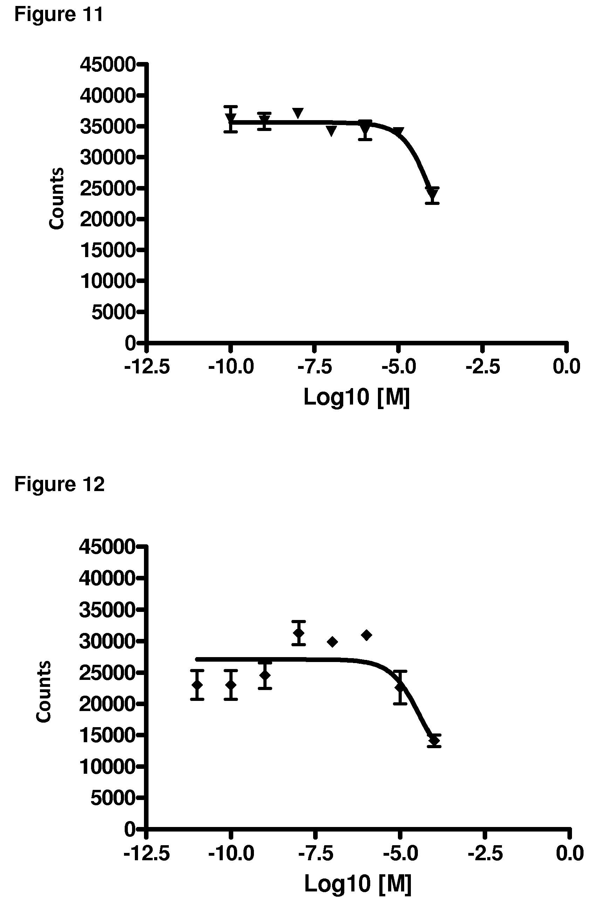

The ligand-receptor assay was performed using the CB₁ labeled antagonist, [³H] SR141716. 450 µL of pH-regulating solution A (50 mM Tris pH = 7.4 with 0.5% bovine serum albumin (BSA)), 100-200 µg rat cerebellum membranes, the diluted product and the labeled CB₁ antagonist, [³H] SR141716, were added in each tube. After incubating for 60 at 37°C, the reaction was stopped with 1 mL of pH-regulating solution A. The mixture was centrifuged at 5000 rpm for 5 minutes. The supernatant was discarded and the pellet washed with another 1 mL of pH-regulating solution A, centrifuged and the supernatant one again was discarded. Scintillation liquid was added and the samples were read in a beta particle counter (Liquid Scintillation Analyzer, Tri-Carb 2100 TR, PACKARD, Packard Bioscience Company). All the products were diluted in pH-regulating solution B (50 mM Tris pH = 7.4 with 0.5% 10 bovine serum albumin (BSA) and 0.3% DMSO) at the concentrations of 10⁻⁵, 10⁻⁶, 10⁻⁷, 10⁻¹, 10⁻¹, 10⁻¹⁰ and 10⁻¹¹ M. All the concentrations of each product were read in triplicate (Figures 9 to 12). The calculated values of Ki and pKi of the assayed compounds are shown in Table 1.

**Table 1**

| Example | CB₁ Kᵢ (M) | CB₁ pKᵢ |
|---|---|---|
| SR141716 | 1.15E-09 | 8.94 |
| Anandamide | 1.7E-07^{a} | 6.55^{a} |
| Win55212-2 | 1.11E-08^{a} | 7.95^{a} |
| 3 | No affinity | No affinity |
| 2 | No affinity | No affinity |
| 5 | 2.25E-05 | 4.65 |
| 10 | 7.49E-06 | 5.12 |

| | | |
|---|---|---|
| ^{a} values listed in the literature (Brit. J. Pharmacol. 1999 128:684) | | |

### Example 14

### In vivo experiments

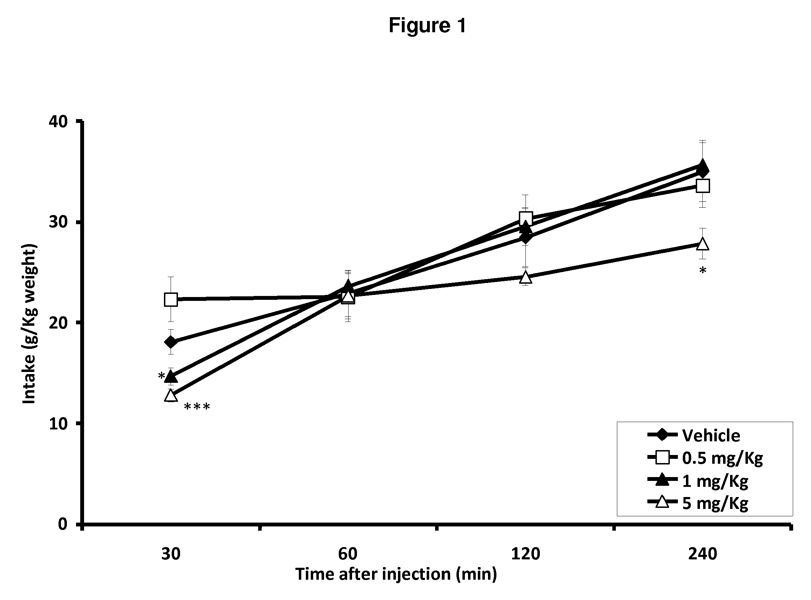

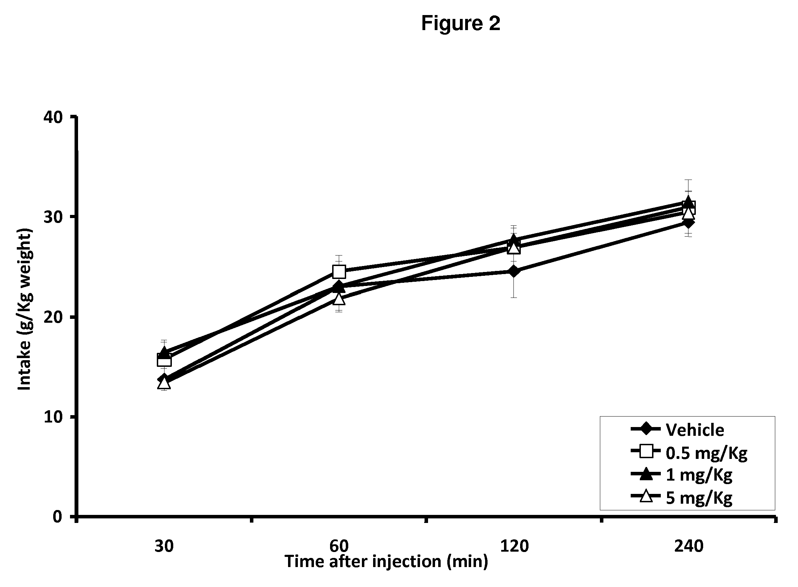

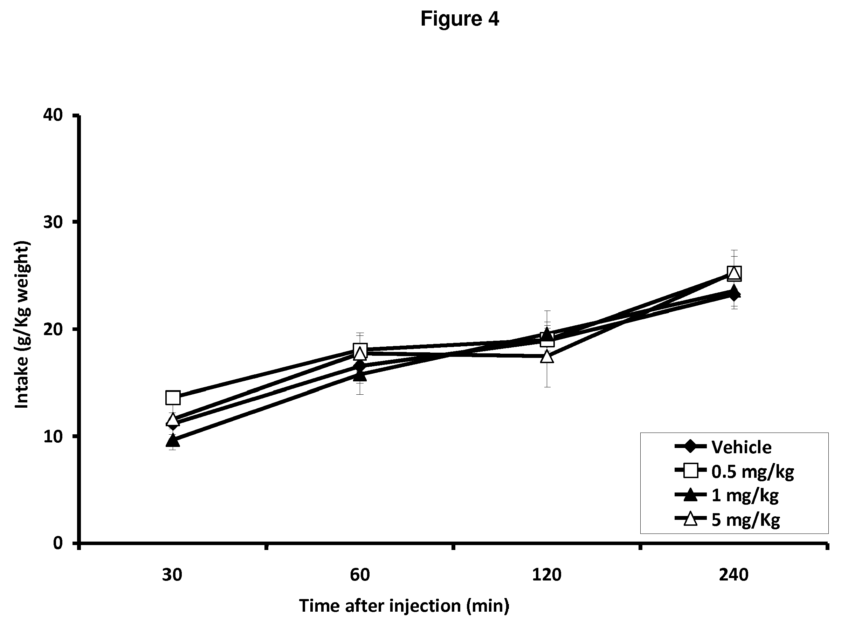

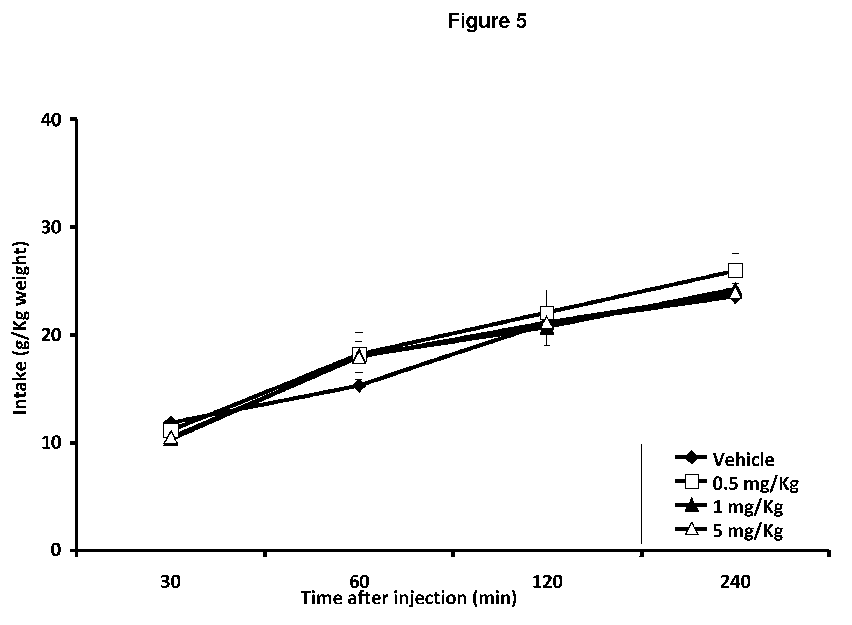

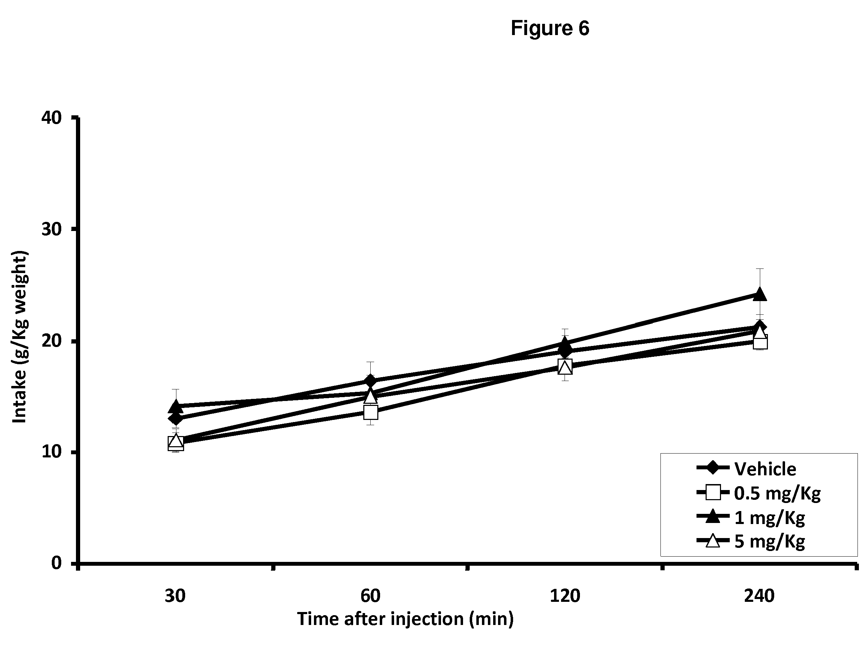

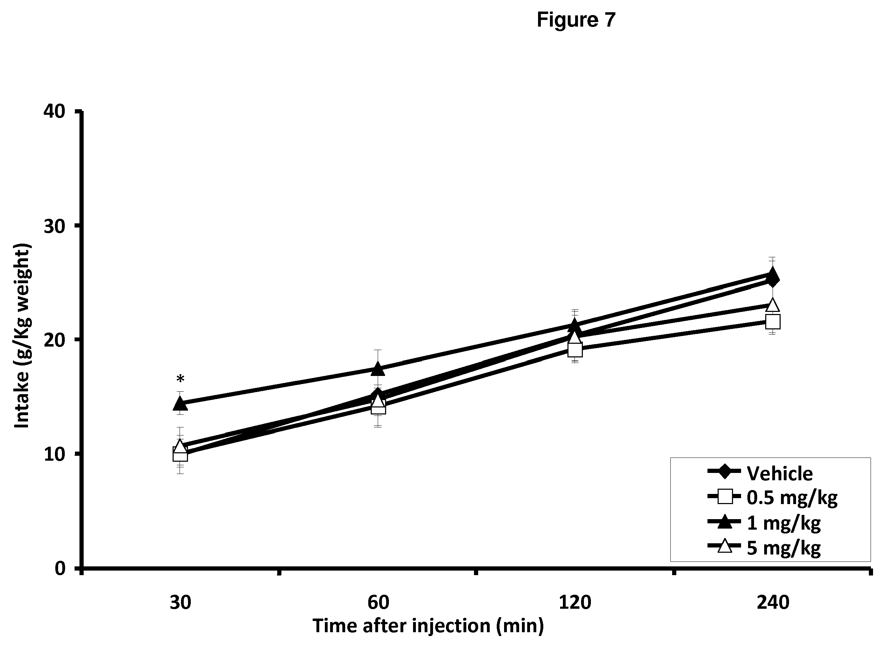

All the *in vivo* experiments were performed using male Wistar rats weighing 200-500 g. The animals were housed in individual cages in a room with controlled temperature (23°C) and humidity (50%) with a 12/12 light and dark cycle. The animals had access to water and food *ad libitum* except in specific experimental methods. The animals were manipulated once a day for the two days before the experimental sessions. All the products were dissolved in a mixture of DMSO (5%), Tween 60 (5%) / saline (90%). The *in vivo* experiments included the analysis of intake and effects on general behavior. None of the assayed compounds altered the general behavior of the animal but some reduced the animal's appetite (Figures 1 to 8).

### Example 15

### Intake experiment

The acute effect on the intake of all the products was tested in animals fasting for 24 hours.

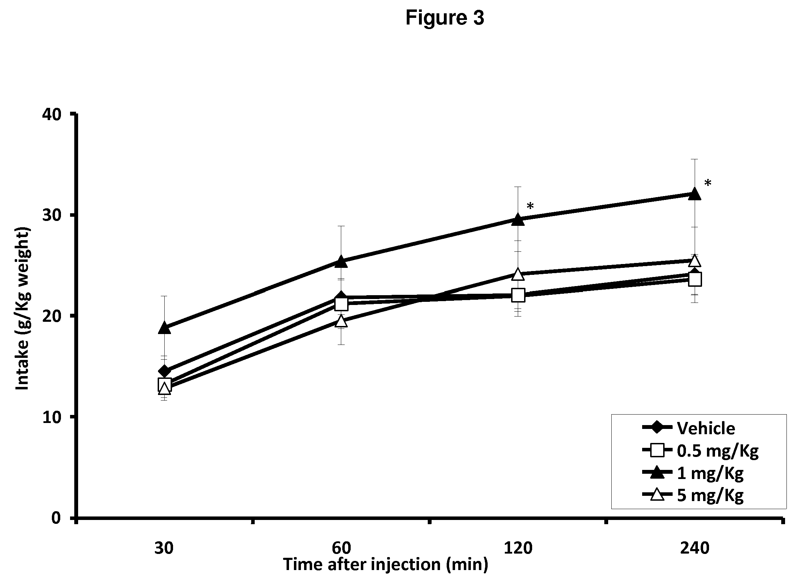

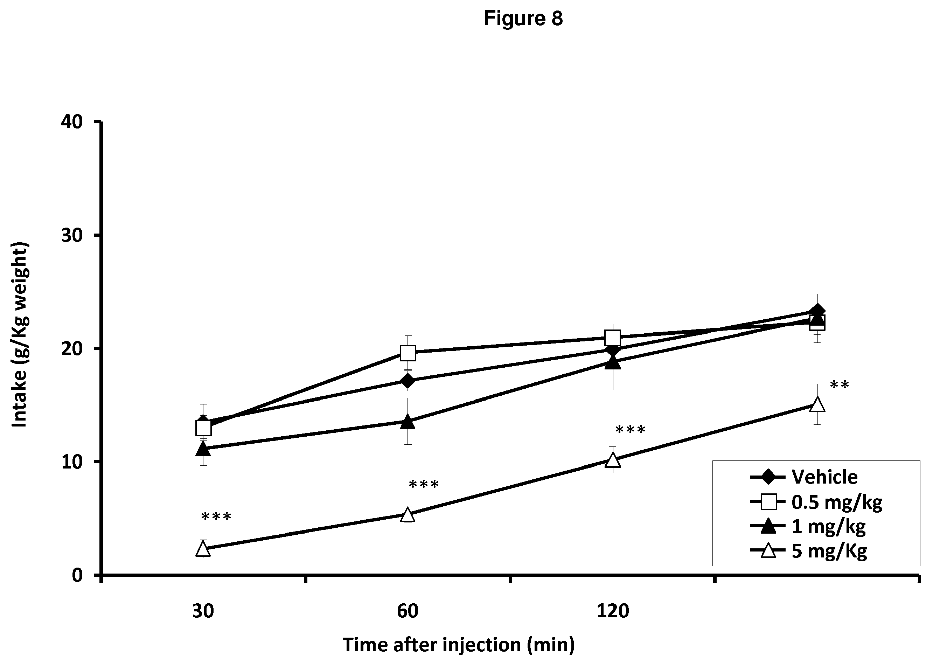

Thirty minutes after injection, previously weighed food was put in the cage. The food was weighed 30, 60, 120 and 240 minutes after starting the test. All the intake experiments were performed with groups of 8 animals (n=8) (Figures 1 to 8). Some of the products of this invention suppressed food intake in animals subjected to a 24-hour fast. The compound (9*Z*,12*Z*)-1-(2-(3,4-dihydroxyphenyl)ethoxy)-octadeca-9,12-diene (Example 10) was the most potent (Figure 8), and its administration reduced intake by 50% with respect to the control group. The compounds (*Z*)-1-(2-phenylethoxy)octadec-9-ene (Example 3, Figure 1) and ((*Z*)-1-(2-(3,4-methylenedioxyphenyl) ethoxy)octadec-9-ene (Example 2, Figure 3) reduced intake by about 25%. All the compounds had long-term effects, the reduction of intake being significant 4 hours after administration.

### Example 16

### Conjugated dienes experiment

### Isolating LDL

Blood from healthy, fasting volunteers is collected in tubes containing 1 g/L of EDTA. The plasma is prepared by centrifugation at 1,000 g and 4°C for 15 minutes. LDL is isolated by sequential ultracentrifugation. Native LDL is dialyzed by size exclusion chromatography in Sephadex G25 columns (Pharmacia, Uppsala, Sweden), with 2.7 ml of 0.01 mol pH 7.4 phosphate buffer (PB) at 4°C. The apolipoprotein B100 content is determined by immunoturbidimetry(ABX Diagnostics - Montpellier, France).

### Monitoring the formation of conjugated dienes

Dialyzed LDL (final concentration of 0.06 g of Apo-B/L) in PB at a final volume of 150 µL was incubated in a 96-well ELISA plate (flat bottom, transparent to UV, Corning®) with 10 µL of methanol in the presence or absence (control) of the compounds object of the study. 10 µL of a 100 µM copper sulfate solution (final concentration of 0.67 µmol) are then added. To minimize evaporation during prolonged incubation periods, 10 µL of mineral oil (Sigma-Aldrich) are added on the surface of the reaction mixture. The plate is covered with a transparent self-adhesive film. The absorbance at 234 nm is continuously monitored every 15 minutes for 24 hours in an Infinite M200 plate reader (TECAN IBERICA, Männedorf, Switzerland). The controls and the samples (containing concentrations of the compounds to be assayed of 0.5, 1 and 3 µM) were evaluated in the same assay in duplicate and each experiment was repeated three times.

### LDL antioxidant activity

In the evaluation of the antioxidant activity of the compounds object of the assay on copper-induced LDL oxidation, hydroxytyrosol (a potent natural antioxidant), tyrosol and homovanillyl alcohol were used as reference compounds. The lag-time (latent period until the formation of conjugated dienes starts) and the ratios between those observed with the compounds object of the assay and that of the native LDL (without added compound), were evaluated. The compound (9*Z*,12*Z*)-1-(2-(3,4-dihydroxyphenyl)ethoxy)-octadeca-9,12-diene of Example 10 showed an activity similar to that of hydroxytyrosol at the three assayed concentrations. The rest of the compounds were only active at the concentration of 3 µM.

## Claims

1. A compound of formula I: 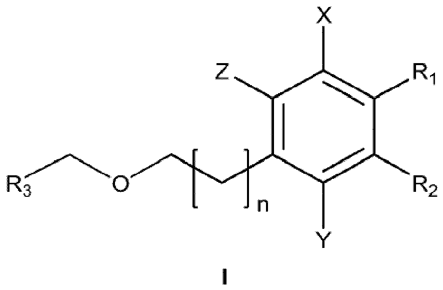 or a salt thereof, where:
X, Y and Z each independently represent hydrogen, halogen, C₁-C₆ alkyl or C₂-C₆ alkenyl, where the C₁-C₆ alkyl and C₂-C₆ alkenyl groups are optionally substituted with one or more R₄ groups;
n represents from 1 to 4;
R₁ and R₂ each independently represent hydrogen or -OR₅;
R₃ represents C₈-C₃₀ alkenyl or C₈-C₃₀ alkynyl;
each R₄ independently represents halogen, -NO₂, -CN, -C₁-C₄ alkoxyl, -NR₆R₆, -NR₆COR₆, -NR₆CONR₆R₆, -NR₆CO₂R₆, -NR₆SO₂R₆, -OR₆,-OCOR₆, -OCONR₆R₆, -OCO₂R₆, -SR₆, -SOR₆, -SO₂R₆, -SO₂NR₆R₆, -SO₂NR₆COR₆, -COR₆, -CO₂R₆ or -CONR₆R₆;
each R₅ independently represents hydrogen or C₁-C₆ alkyl;
or when R₁ and R₂ simultaneously represent -OR₅, the two R₅ groups are optionally bound forming a group of formula -O-W-O-;
W represents C₁-C₄ alkylenyl optionally substituted with one or
more C₁-C₄ alkyl, =O, =NR₆ or =S; and
each R₆ independently represents hydrogen or C₁-C₄ alkyl,
on the proviso that the compound (9*Z*,12*Z*)-1-(2-(3,4-methylenedioxyphenyl)ethoxy)-octadeca-9,12-diene is excluded.

2. The compound according to claim 1, where X, Y and Z independently represent hydrogen, halogen or C₁-C₆ alkyl, where the C₁-C₆ alkyl group is optionally substituted with one or more R₄ groups.

3. The compound according to any of claims 1 or 2, where X and Y independently represent hydrogen.

4. The compound according to any of claims 1 to 3, where Z represents hydrogen.

5. The compound according to any of claims 1 to 4, where n represents 1.

6. The compound according to any of claims 1 to 5, where R₁ and R₂ each independently represents hydrogen.

7. The compound according to any of claims 1 to 5, where R₁ and R₂ each independently represents -OR₅.

8. The compound according to claim 7, where R₁ and R₂ simultaneously represent -OR₅.

9. The compound according to claim 8, where when R₁ and R₂ simultaneously represent -OR₅, the two R₅ groups are bound forming a group of formula -O-W-O-.

10. The compound according to claim 9, where W represents C₁-C₄ alkylenyl optionally substituted with one or more C₁-C₄ alkyl.

11. The compound according to claim 10 of formula III: 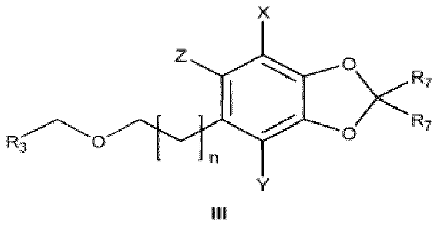 where each R₇ independently represents C₁-C₄ alkyl, preferably methyl.

12. The compound according to any of claims 1 to 5, where:
R₁ represents hydrogen; and
R₂ represents -OR₅.

13. The compound according to any of claims 1 to 5, where:
R₁ represents -OR₅; and
R₂ represents hydrogen.

14. The compound according to any of claims 1 to 13, where R₃ represents C₈-C₃₀ alkenyl.

15. The compound according to any of claims 1 to 14, where each R₄ independently represents halogen, -C₁-C₄ alkoxyl, -NR₆R₆,-OR₆, -SR₆, -SOR₆, -SO₂R₆, -COR₆, -CO₂R₆ or -CONR₆R₆; preferably halogen, -C₁-C₄ alkoxyl, -NR₆R₆, -OR₆, -SR₆ or -COR₆.

16. The compound according to any of claims 1 to 5 and 7 to 15, where each R₅ independently represents hydrogen.

17. The compound according to claim 1 selected from:
(*Z*)-1-(2-phenylethoxy)octadec-9-ene;
(*Z*)-1-(2-(4-hydroxyphenyl)ethoxy)octadec-9-ene;
(*Z*)-1-(2-(3,4-methylenedioxyphenyl)ethoxy)octadec-9-ene;
(*Z*)-1-(2-(3,4-dihydroxyphenyl)ethoxy)octadec-9-ene;
(9*Z*,12*Z*)-1-(2-phenylethoxy)-octadeca-9,12-diene;
(9*Z*,12*Z*)-1-(2-(4-hydroxyphenyl)ethoxy)-octadeca-9,12-diene; and
(9*Z*,12*Z*)-1-(2-(3,4-dihydroxyphenyl)ethoxy)-octadeca-9,12-diene.

18. A pharmaceutical composition comprising at least one of the compounds of formula I as defined in claims 1 to 17, or a salt thereof, and at least one pharmaceutically acceptable carrier, adjuvant and/or vehicle.

19. The pharmaceutical composition according to claim 18, further comprising another active ingredient.

20. Use of a compound of formula II: 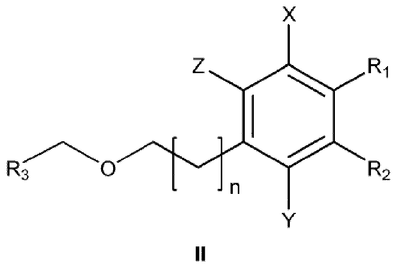 or a salt thereof, where:
each independent X, Y and Z represents hydrogen, halogen, C₁-C₆ alkyl or C₂-C₆ alkenyl, where the C₁-C₆ alkyl and C₂-C₆ alkenyl groups are optionally substituted with one or more R₄ groups;
n represents from 1 to 4;
R₁ and R₂ each independently represent hydrogen or -OR₅;
R₃ represents C₈-C₃₀ alkenyl or C₈-C₃₀ alkynyl;
each R₄ independently represents halogen, -NO₂, -CN, -C₁-C₄ alkoxyl, -NR₆R₆, -NR₆COR₆, -NR₆CONR₆R₆, -NR₆CO₂R₆, -NR₆SO₂R₆, -OR₆,-OCOR₆, -OCONR₆R₆, -OCO₂R₆, -SR₆, -SOR₆, -SO₂R₆, -SO₂NR₆R₆, -SO₂NR₆COR₆, -COR₆, -CO₂R₆ or -CONR₆R₆;
each R₅ independently represents hydrogen or C₁-C₆ alkyl;
or when R₁ and R₂ simultaneously represent -OR₅, the two R₅ groups are optionally bound forming a group of formula -O-W-O-;
W represents C₁-C₄ alkylenyl optionally substituted with one or
more C₁-C₄ alkyl, =O, =NR₆ or =S; and
each R₆ independently represents hydrogen or C₁-C₄ alkyl,
for manufacturing a medicament.

21. Use of a compound of formula II as defined in claim 20 for manufacturing a medicament for the treatment and/or prevention of an eating disorder disease.

22. Use according to claim 21 for the treatment and/or prevention of a disease selected from obesity, lipid dysfunction, diabetes, cardiovascular diseases and metabolic syndrome.

23. Use according to any of claims 21 or 22 to reduce subcutaneous fat and/or to induce satiety and control intake.

24. Use of a compound of formula II as defined in claim 20 for manufacturing a medicament for the treatment and/or prevention of LDL oxidation.

25. Use of a compound of formula II as defined in claim 20 for manufacturing a medicament for the treatment and/or prevention of a disease associated with LDL oxidation

26. Use of a compound of formula II as defined in claim 20 for manufacturing a medicament for the treatment and/or prevention of arteriosclerosis.

27. A method for preparing a compound of formula I as defined in claims 1 to 17 comprising:
a) reacting a compound of formula IV with a compound of formula V: 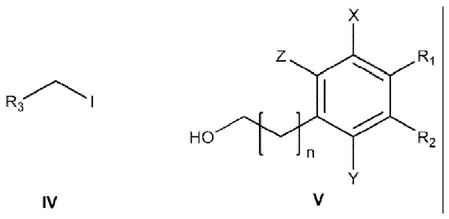 where X, Y, Z, R₁, R₂, R₃ and n have the previously described meaning; or
b) converting a compound of formula I into another compound of formula I in one or several steps.
